## Europäisches Patentamt

## European Patent Office

## Office européen des brevets

(19)

(11) Veröffentlichungsnummer: **0 099 084**
**B1**

(12) # EUROPÄISCHE PATENTSCHRIFT

(45) Veröffentlichungstag der Patentschrift:
31.05.89

(21) Anmeldenummer: **83106730.1**

(22) Anmeldetag: **08.07.83**

(51) Int. Cl.⁴: **C 12 N 15/00,** C 12 P 21/02,
C 07 K 13/00, C 07 H 21/04,
C 12 N 1/20 //
C12R1/19

(54) Verfahren zur Herstellung von Interferon und Expressionsvektoren dafür.

(30) Priorität: **12.07.82 US 397388**

(43) Veröffentlichungstag der Anmeldung:
**25.01.84 Patentblatt 84/4**

(45) Bekanntmachung des Hinweises auf die Patenterteilung:
**31.05.89 Patentblatt 89/22**

(84) Benannte Vertragsstaaten:
**AT BE CH DE FR GB IT LI NL**

(56) Entgegenhaltungen:
**EP-A-0 041 767**
**EP-A-0 052 002**
**EP-A-0 089 676**

**NATURE, Band 295, 11. Februar 1982, Seiten 503-508;
P.W. GRAY et al.: "Expression of human immune
interferon cDNA in E. coli and monkey cells"
CHEMICAL ABSTRACTS, Band 96, Nr. 21, 24. Mai
1982, Seite 174, Nr. 175316s, Columbus, Ohio, US;
H.M. SHEPARD et al.: "Increased synthesis in E.
coli of fibroblast and leukocyte interferons through
alterations in ribosome binding sites"**

(73) Patentinhaber: **F. HOFFMANN- LA ROCHE & CO.
Aktiengesellschaft, CH- 4002 Basel (CH)**

(72) Erfinder: **Crowl, Robert M., 246 Paterson Avenue,
Little Falls, N.J. (US)**

(74) Vertreter: **Lederer, Franz, Dr., Van der Werth,
Lederer & Riederer Patentanwälte Lucile- Grahn-
Strasse 22, D-8000 München 80 (DE)**

**Beschreibung**

Gegenwärtig ist bekannt, daß verschiedene Faktoren die Expressionsleistung eukaryotischer Proteine durch transformierte Mikroorganismen beeinflussen. Zwei der bedeutendsten Faktoren, die diese Proteinexpression sowohl definieren als auch steuern, sind die Genauigkeit, mit der die Bakterienzelle das eingesetzte Gen in mRNA transkribiert, und die Translationsleistung mit der diese mRNA durch bakterielle Ribosomen in Protein übersetzt wird. Die Transkriptions- und Translationsleistung, allgemein als Expression bekannt, hängt, wie angenommen wird, von den Nukleotidsequenzen ab, die typischerweise vor den klonierten Genen auf der Vektor-DNA liegen. Diese Expressionskontrollsequenzen umfassen großenteils Promotor/Operator-Stellen, mit denen die RNA-Polymerase eine Wechselwirkung eingeht, um Transkription zu initiieren, und Ribosomenbindungsstellen (RBS), an die die Ribosomen binden und mit ihnen in Wechselwirkung treten, um die Translation in Protein zu initiieren.

Verschiedene Expressionskontrollsequenzen, die sich von den natürlicherweise in Plasmiden auftretenden unterscheiden, sind nach dem Stand der Technik zur Verbesserung der Kontrolle über die Expression eukaryotischer Proteine und Polypeptide in Bakterienwirten eingesetzt worden. Diese umfassen z. B. die Nutzung des Operators, Promotors, Ribosomenbindungs- und Wechselwirkungssequenzen des Lactose-Operons von E. coli, die entsprechenden Sequenzen des Tryptophansynthetase-Systems von E. coli und die Hauptoperator- und Promotorbereiche des Bakteriophagen λ (H. Bernard et al., Gene 5, (1979) 59 - 76).

Der $P_L$-Promotor des λ-Phagen wurde zur Expression eukaryotischer und prokaryotischer, in einen prokaryotischen Organismus klonierter Genprodukte verwendet. Die Promotoraktivität von $P_L$ erwies sich bei tiefer Temperatur in Gegenwart eines temperaturempfindlichen Gens, das einen temperaturempfindlichen Repressor spezifiziert, als abgeschaltet, konnte aber durch Wärmeinduktion bei Expression eines prokaryotischen Proteins aktiviert werden. Beispiele des Standes der Technik, die dieses Promotorsystem nutzen, sind die Europäische Patentanmeldung 81 301 413.1 vom 1.4.1981 und der Artikel von Derynck et al. in Nature 287, (1980) 193 - 197. Gemäss diesen Veröffentlichungen wurde das $P_L$-Promotorsystem in Verbindung mit der ganzen bakteriellen Ribosomenbindungsstelle zur Expression eukaryotischer Proteine, wie Fibroblasten-Interferon, verwendet.

Zur Expression eukaryotischer Gene in E. coli sind zwei verschiedene Lösungen herangezogen worden, die beide rekombinante DNA-Techniken anwenden, um das Fehlen geeigneter Regelsignale vor den eukaryotischen Genen zu überwinden:

(1) Insertion der das eukaryotische Gen kodierenden Sequenz in den kodierenden Bereich eines bakteriellen Gens, z. B. das β-Lactamase-Gen, womit die gesamte RBS des E. coli-Gens genutzt wird, oder

(2) der Aufbau einer sogenannten "Hybrid"-RBS, bestehend aus einer Shine-Dalgarno (SD)-Sequenz, den Sequenzen proximal (oberhalb in 5'-Richtung) zur SD-Sequenz (von E. coli-DNA) und dem AUG-Startkodon plus restlicher Kodierungssequenz aus der eukaryotischen DNA. Die SD-Sequenz ist eine Sequenz von 3 bis 9 Basen, die komplementär zu Basen am 3'-Ende von 16 s-ribosomaler RNA sind und zwischen dem Promotor und dem Initiationskodon für das eukaryotische Gen liegen.

Der Hauptnachteil der ersten Lösung liegt darin, daß sie zur Produktion eines Fusions-Polypeptids führt, d.h., ein Teil des Genprodukts ist durch die E. coli-DNA und ein Teil durch die eukaryotische DNA kodiert. Die zweite Lösung ist erfolgreich angewandt worden, um authentische, reife Genprodukte (vollständig und biologisch aktiv, ohne Präsequenz) zu produzieren, die identisch sind mit den natürlichen, d.h mit den in der eukaryotischen Zelle produzierten (Derom et al., Gene 17, (1982) 45 - 54; und Gheysen et al., Gene 17, (1982) 55 - 63).

Bisher ist die Expression verschiedener Human-Interferon-Spezies, wie Leukozyten-, Fibroblasten- und Immun-Interferon, mit beiden Lösungen versucht worden. Beim Versuch der Expression von Fibroblasten-Interferon (IFN-β) wandten Gheysen et al. (s.o.) die erste Lösung (vollständige bakterielle RBS) in Verbindung mit dem $P_L$-Promotor-System an. Obgleich diese Veröffentlichung angibt, daß biologisch aktive, nicht fusionierte Proteine erhalten wurden, ist klar, daß dies ein Ergebnis des Prozessierens von Fusions-IFN-β-Proteinen nach unbekannten und unzulänglichen Mechanismen innerhalb der Zelle war, da das IFN-β-Gen nicht die geeigneten Translationsinitiationssequenzen besitzt. Aus dieser Literatur ergibt sich ferner, daß das eukaryotische Genprodukt auch die durch den Signalteil des eukaryotischen Gens kodierte Aminosäuresequenz enthält. Daher wird nicht reifes Interferon exprimiert.

In den Veröffentlichungen von Gray et al., Nature 295, (1982) 503 - 508, und Shepard et al., DNA 1, (1982) 125 - 131 wurde die zweite Lösung, die "Hybrid-RBS", angewandt. Doch erfolgte die Expression unter der Kontrolle des E. coli-trp-Promotors, und die Hybrid-RBS stammte von dem ATG des eukaryotischen Gens, der SD- und der Verknüpfungssequenz mit den trp-Leader. Ferner blieben gemäss diesen beiden Veröffentlichungen die SD-Sequenz und die Sequenz proximal (flussaufwärts in 5'-Richtung) zur SD-Sequenz unverändert. Die einzige eingeführte Variabilität, d.h. eine Änderung sowohl in der Zahl als auch in der Zusammensetzung der Nukleotide zwischen der SD-Sequenz und dem ATG-Kodon, erfolgte im Verknüpfungsbereich.

Auch in der Europäischen Patentanmeldung 81 301 413.1 (s.o.) wurde diese zweite Lösung, die "Hybrid-RBS", angewandt. Doch blieb auch hier die SD-Sequenz und die Sequenz proximal zur SD-Sequenz unverändert. Die einzige eingeführte Variabilität erfolgte im Verknüpfungsbereich durch Verknüpfung der $P_L$-Promoter und $O_L$-Operator-Sequenz mit einer von Bakteriophagen MS2 stammenden natürlichen Ribosomenbindungsstelle.

Dem Stand der Technik fehlte eine Massnahme zur Gestaltung und zum Aufbau einer hybriden Ribosomenbindungsstelle mit der Flexibilität, die erforderlich ist, um die Expression eines klonierten

EP 0 099 084 B1

eukaryotischen Gens selektiv zu steigern. Ferner fehlte dem Stand der Technik eine Maßnahme zum Steuern der Expression eines klonierten eukaryotischen Genprodukts in seiner reifen Form, mit einem temperaturempfindlichen Promotorsystem und ohne die Notwendigkeit weiterer Aufbereitung von Fusionsproteinen in der Zelle.

Die Erfindung überwindet die vorgenannten Einschränkungen des Standes der Technik durch Schaffung verbesserter Vektoren und Verfahren zum Steuern der Expression klonierter Gene, die reife Interferone kodieren, wie reifes Human-Leukozyten-, -Fibroblasten- und -Immun-Interferon, in bakteriellen Wirten.

Im weitesten Sinne umfasst die Erfindung Massnahmen bzw. Mittel zum Steuern der Expression von reifen Human-Leukozyten-, -Fibroblasten- und -Immun-Interferon-Proteinen, wobei ein prokaryotischer Organismus versehen wird mit den $P_L$-Promotor- und $O_L$-Operatorgenen, isoliert vom Bakteriophagen $\lambda$, einem weiteren Gen, cl, isoliert vom Lambda-Phagen, das ein temperaturempfindliches (ts) Repressorprotein kodiert, und einer hybriden Ribosomenbindungsstelle, verbunden sowohl mit den Lambda-Phagen $P_L$ Promotor/$O_L$ Operator Sequenzen und einem eukaryotischen Gen, das das reife Humaninterferon kodiert. Die vorliegende Erfindung umfaßt auch eine Lyseverfahren zur Öffnung der Mikrobenzellwände zwecks Freisetzung des reifen eukaryotischen Proteins, welches durch den Organismus ohne Beeinträchtigung der Aktivität des reifen Proteins exprimiert wird.

Fig. 1    ist eine partielle Restriktionskarte eines 1200 BP BglII-BamHI-Fragments, das den $P_L$-Promotor enthält;
Fig. 2    veranschaulicht die Konstruktion des Expressionsvektors pRC14 der den Lambda-$P_L$-Promotor auf einem 350 BP-Insert enthält;
Fig. 3    veranschaulicht den Einbau des Leukozyten-Interferongens in den Expressionsvektor pRC14;
Fig. 4    veranschaulicht die Isolierung des 82BP-Fragments, welches die SD-Sequenz des int-Gens des Bakteriophagen Lambda enthält und welches zum Aufbau des Expressionsvektors pRC15 verwendet wurde;
Fig. 5    veranschaulicht die Konstruktion des Expressionsvektors pRC15;
Fig. 6    veranschaulicht die Konstruktion der Expressionsvektoren pRC21 und pRC22 aus den Plasmiden pRC14 bzw. pRC15;
Fig. 7    veranschaulicht die Konstruktion des Expressionsvektors pRC23 mit einem $P_L$-Promotor und einer synthetischen RBS, die aus dem Plasmid pRC2 stammen;
Fig. 8    zeigt das Schema, das zum Einsetzen des Fibroblasteninterferon kodierenden Gens in die Plasmide pRC21 und pRC22 angewandt wurde, und die Sequenzen der erhaltenen Promotor-Genverbindungen;
Fig. 9    zeigt die Restriktionsenzym-Spaltungskarte des Plasmids bHIT3709, wie in Beispiel 5 (vii) erhalten, wobei der FIGFIG -Teil die Sequenz anzeigt, die das Peptid kodiert, das vermutlich das Signalpeptid ist, und der FIGFIG -Teil die Sequenz angibt, die das IFI-Polypeptid kodiert;
Fig. 10   zeigt die Basensequenz des Plasmids pHIT3709, wie in Beispiel 5 (vii) erhalten;
Fig. 11   zeigt das Schema, das angewandt wurde, um das Gen, das Immun-Interferon kodiert, in den Expressionsvektor pRC22 einzufügen, und die Sequenz der Promotor-Gen-Verbindung;
Fig. 12   veranschaulicht die Insertion des Immun-Interferongens in den Expressionsvektor pRC23 und die Sequenz der Promotor-Gen-Verbindung sowie ihre Abwandlung.

Die Erfindung schafft Expressionsvektoren, die Interferon zu exprimieren vermögen, vorzugsweise reifes Human-Immun-Interferon, enthaltend eine $P_L$ Promotor- und $O_L$ Operator-DNA-Sequenz, die aus dem Bakteriophagen Lambda stammt, eine DNA-Sequenz, die eine Hybridribosomenbindungsstelle kodiert, und eine DNA-Sequenz, die reifes Interferon kodiert, vorzugsweise reifes Human Immun Interferon. Die Erfindung umfaßt ferner Organismen, vorzugsweise Bakterien, wie Escherichia coli, die mit diesen Vektoren transformiert sind. Die Erfindung umfaßt schließlich ein Verfahren zur Herstellung von reifem Human-Interferon, dadurch gekennzeichnet, dass man

a)    einen Wirtsorganismus mit einem Expressionsvektor, wie oben definiert, transformiert.
b)    den transformierten Organismus züchtet, wodurch er das durch den Expressionsvektor der Stufe a) kodierte reife Human-Interferon produziert;
c)    den Organismus lysiert und
d)    das besagte Interferon aus dem anfallenden Lysat isoliert.

In einer bevorzugten Ausführungsform der Erfindung enthält entweder der Expressionsvektor oder der Wirtsorganismus auf einem Chromosom ein mutantes cl-Repressorgen, das aus einem $\lambda$-Bakteriophagen stammt und ein temperaturempfindliches Repressorprotein kodiert. Alternativ kann bei dem Verfahren zur Herstellung von reifem Human-Interferon, wie oben definiert, der Wirtsorganismus mit einem solchen mutanten Repressorgen durch Transformation mit einem separaten, replizierbaren Vektor versehen werden, der solch ein Gen zu exprimieren vermag und mit dem Expressionsvektor der Stufe (a) voll kompatibel ist.

Nach Stufe (b) des obigen Verfahrens werden die Zellen nach einem bekannten Verfahren gesammelt und nach dem Suspendieren in einer Pufferlösung lysiert. Der Begriff Lyse, wie er hier verwendet wird, soll eine Arbeitsweise zum Öffnen von Zellwänden im Wirt bedeuten, die vorzugsweise enzymatisch erfolgt, obgleich es auch mit Ultraschall, mechanisch oder nach irgend einer anderen bekannten und vom Fachmann angewandten Maßnahme geschehen kann. Das Interferon kann aus dem Lysat durch dem Fachmann auf dem Gebiet

3

bekannten Proteinreinigungsverfahren gewonnen werden, z. B. durch Elektrophorese oder Chromatographie (z. B. Antikörper-Affinitätschromatographie). Das erfindungsgemäß bevorzugte Gen kodiert Human-Immun-Interferon in reifer Form, d.h. ohne irgendwelche Präsequenz.

Die in dieser Beschreibung und den Ansprüchen verwendeten Abkürzungen und Ausdrücke sind, sofern nicht im einzelnen erklärt, solche, wie sie üblicherweise dem Fachmann bekannt sind und von ihm verwendet werden.

Das erfindungsgemäße reife Human-Immun-Interferon wird durch die folgende DNA-Sequenz kodiert:

```
(5') TGT TAC TGC CAG GAC CCA TAT GTA AAA GAA
     GCA GAA AAC CTT AAG AAA TAT TTT AAT GCA GGT
     CAT TCA GAT GTA GCG GAT AAT GGA ACT CTT TTC
     TTA GGC ATT TTG AAG AAT TGG AAA GAG GAG AGT
     GAC AGA AAA ATA ATG CAG AGC CAA ATT GTC TCC
     TTT TAC TTC AAA CTT TTT AAA AAC TTT AAA GAT
     GAC CAG AGC ATC CAA AAG AGT GTG GAG ACC ATC
     AAG GAA GAC ATG AAT GTC AAG TTT TTC AAT AGC
     AAC AAA AAG AAA CGA GAT GAC TTC GAA AAG CTG
     ACT AAT TAT TCG GTA ACT GAC TTG AAT GTC CAA
     CGC AAA GCA ATA CAT GAA CTC ATC CAA GTG ATG
     GCT GAA CTG TCG CCA GCA GCT AAA ACA GGG AAG
     CGA AAA AGG AGT CAG ATG CTG TTT CGA GGT CGA
     AGA GCA TCC CAG -X (3')                              (I)
```

worin X TAA, TGA oder TAG ist.

Am 5'-Ende der obigen DNA-Sequenz kann die Sequenz 5'ATG AAA TAT ACA AGT TAT ATC TTG GCT TTT CAG CTC TGC ATC GTT TTG GGT TCT CTT GGC 3' (III) oder ein ATG-Triplett vorliegen, das Methionin kodiert und somit auch Immun-Interferon mit der Vorsequenz Met-Lys-Tyr-Thr-Ser-Tyr-Ile-Leu-Ala-Phe-Gln-Leu-Cys-Ile-Val-Leu-Gly-Ser-Leu-Gly oder mit Met, addiert an das N-Ende, exprimiert. An einem Expressionsvektor ist das die DNA-Sequenz kodierende Immun-Interferon hinter einer adäquaten Promotor-Operator-Sequenz über eine SD-Sequenz verbunden.

Das durch die vorgenannte DNA-Sequenz I kodierte reife Human-Immun-Interferonprotein einschließlich dem ATG-Startsignal ist durch die folgende Aminosäuresequenz charakterisiert:

H₂N- Met-Cys-Tyr-Cys-Gln-Asp-Pro-Tyr-Val-Lys-Glu-Ala-
     Glu-Asn-Leu-Lys-Lys-Tyr-Phe-Asn-Ala-Gly-His-Ser-
     Asp-Val-Ala-Asp-Asn-Gly-Thr-Leu-Phe-Leu-Gly-Ile-
     Leu-Lys-Asn-Trp-Lys-Glu-Glu-Ser-Asp-Arg-Lys-Ile-
     Met-Gln-Ser-Gln-Ile-Val-Ser-Phe-Tyr-Phe-Lys-Leu-
     Phe-Lys-Asn-Phe-Lys-Asp-Asp-Gln-Ser-Ile-Gln-Lys-
     Sdr-Val-Glu-Thr-Ile-Lys-Glu-Asp-Met-Asn-Val-Lys-
     Phe-Phe-Asn-Ser-Asn-Lys-Lys-Lys-Arg-Asp-Asp-Phe-
     Glu-Lys-Leu-Thr-Asn-Tyr-Ser-Val-Thr-Asp-Leu-Asn-
     Val-Gln-Arg-Lys-Ala-Ile-His-Glu-Leu-Ile-Gln-Val-
     Met-Ala-Glu-Leu-Ser-Pro-Ala-Ala-Lys-Thr-Gly-Lys-
     Arg-Lys-Arg-Ser-Gln-Met-Leu-Phe-Arg-Gly-Arg-Arg-
     Ala-Ser-Gln-OH (II)

Es ist auch vorhersehbar, daß bei der praktischen Durchführung der Erfindung die erste Aminosäure, Met, die durch das ATG-Startsignal kodiert wird, durch den Mikroorganismus nach Expression abgespalten werden wird.

Die Aminosäuresequenzen, die reifes Human-IFN-β und IFN-α (mehrere verschiedene Arten) definieren, sowie die entsprechenden DNA-Sequenzen, die sie kodieren, sind veröffentlicht und dem Fachmann wohlbekannt.

Erfindungsgemäß wird die das Interferon kodierende DNA-Sequenz in die DNA eines Plasmids oder Klonungsvektors eingefügt, wodurch eine rekombinante DNA-Sequenz gebildet wird, die dann zum Transformieren einer Wirtszelle in üblicher Weise herangezogen wird. Erfindungsgemäß wird eine transformierte Wirtszelle kloniert, vorzugsweise in vitro.

Die erfindungsgemäßen Expressionsvektoren enthalten ferner Hybrid-RBS-kodierende DNA-Sequenzen Eine RBS, die notwendig ist für die Initiation der Translation in einer Wirtszelle, besteht aus

(1) einem ATG-Translationsinitiationskodon für die Aminosäure Methionin (alle bekannten E coli-Genprodukte beginnen mit der Aminosäure Methionin, die anschließend abgespalten werden kann oder auch nicht),

(2) einer Sequenz von 3 bis 9 Basen, die komplementär zu Basen am 3'-Ende 16s-ribosomaler RNA sind und als SD-Sequenz bekannt sind, und

(3) einer Basensequenz zwischen diesen beiden, bekannt als Verknüpfungsbereich ("linker region").

Beispielsweise ist die Sequenz am Beginn des E. coli-Lac-z-Gens ACAGGAA ACAGCT(ATG-)-. Die unterstrichene Sequenz ist die SD-Sequenz, die vom ATG-Triplett durch 7 Basen getrennt ist. Die Länge des Verknüpfungsbereichs zwischen der SD-Sequenz und dem ATG-Triplett kann von Gen zu Gen von etwa 5 bis 16 Basen variieren, und es scheint, daß eine Korrelation besteht zwischen diesem Abstand und der Höhe der Proteinexpression. Ausserdem kann auch die Sequenz des Verknüpfungsbereichs die Expressionshöhe beträchtlich beeinflussen. Ausserdem wurde gefunden, dass nicht nur die Natur der SD-Sequenz selbst, sondern auch die sie flankierenden DNA-Sequenzen und das ATG-Startkodon für eine wirksame Translation einer mRNA von Bedeutung sind.

Die erfindungsgemäßen Hybrid-RBSs unterscheiden sich von natürlich vorkommenden RBSs und den bekannten Hybrid-RBSs (s.o.) durch Länge der DNA-Sequenz und/oder die Reihenfolge der Basen innerhalb der Sequenz. Die Unterschiede liegen im Verknüpfungsbereich, in der SD-Sequenz oder in der die SD-Sequenz flankierenden Sequenz (nach vorne in 5'-Richtung). Die folgenden Hybrid-RBSs sind von speziellem Interesse:

```
GCTAACTGACAGGAGAATTC
CCTTTTGAAGAGGATCTGAATTC
TTAAAAATTAAGGAGGAATTC
AGGAGAATTCATG
AGGAGAATTAATTCATG
AGGAGAATTCTAGATG
AGGATCTGAATTCTAGATG
AGGAGAATTAATTCTAGATG
AGGATCTGAATTAATTCTAGATG
AGGAGAATTCTAGCTAGATG und
AGGATCTGAATTCTAGCTAGATG.
```

In einer bevorzugten Ausführungsform der Erfindung ist der als Empfänger bei den Transformationen verwendete Mikroorganismus Escherichia coli K-12, Stamm 294, wie in der veröffentlichten Britischen Patentanmeldung 2 055 382A beschrieben. Dieser Mikroorganismus ist bei der American Type Culture Collection, ATCC-Nr. 31446, am 28. Oktober 1978 hinterlegt worden und ist öffentlich zugängig. Ferner wurde die gesamte Arbeit mit rekombinanter DNA in Übereinstimmung mit den Richtlinien des National Institutes of Health (NIH) durchgeführt.

Obgleich die Erfindung in den am meisten bevorzugten Ausführungsformen an E. coli K-12, Stamm 294, beschrieben wird, können andere bekannte Stämme von E. coli, z. B. E. coli MA210 oder RR1, oder andere Mikrobenstämme, die hinterlegt und von anerkannten Hinterlegungsstellen für Mikroorganismen, wie der American Type Culture Collection (ATCC), erhältlich sind, bei der Durchführung der vorliegenden Erfindung verwendet werden.

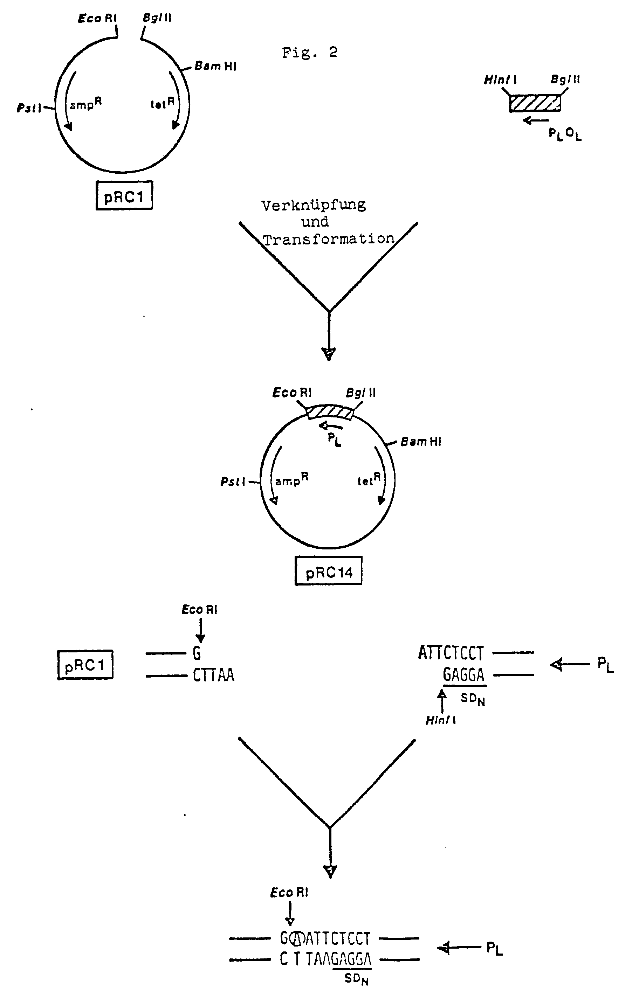

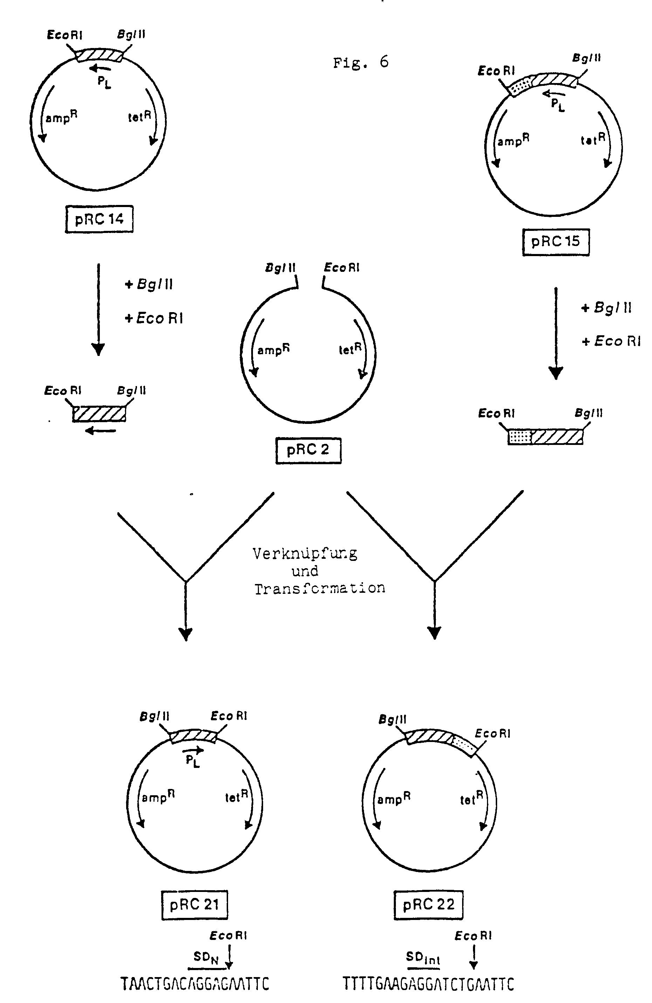

Die erfindungsgemäßen Expressionsvektoren sind Derivate des Plasmids pBR322 (s. Fig. 2, 5, 6 und 7), enthaltend den $P_L$-Promotor, isoliert aus Bakteriophagen λ-DNA und eingeführt zwischen den $tet^R$- und $amp^R$-Genen, orientiert in beiden Richtungen (d.h. die Transkription läuft ab gegen das $amp^R$- oder gegen das $tet^R$-Gen). $P_L$ war der Promotor der Wahl, da es ein sehr starker Promotor ist, der durch den λ cl-Repressor wirksam und bequem gesteuert werden kann. Das den Repressor kodierende Gen trägt eine Mutation, clts2 oder clts857, die den Repressor temperaturempfindlich macht. Bei 30°C wirkt der Repressor normal, und von etwa 37°C bis etwa 42°C wird er inaktiviert. So wird der $P_L$-Promotor bei 30°C reprimiert (abgeschaltet) und bei 42°C dereprimiert (eingeschaltet). Dieses Merkmal ist hier wünschenswert, da das interessierende Genprodukt für die Zelle toxisch oder, wenn in großer Menge vorhanden, für das Zellwachstum schädlich sein kann. Die Fähigkeit zur Kontrolle des $P_L$-Promotors ermöglicht es, die Kultur ohne Expression des Genprodukts bei etwa 30°C bis etwa 36°C zu züchten und nacn einer optimalen Zeit die Temperatur von etwa 37 auf etwa 42°C anzuheben, um das gewünschte Genprodukt zu produzieren.

Alle Vektoren der offenbarten Erfindung enthalten auch eine EcoR1-Restriktionsstelle in 3'-Richtung hinter (distal) der SD-Sequenz und im Abstand von 0, 1 oder 4 Basen, wodurch ein Mittel zum Aufbau verschiedener Hybrid-RBSs geschaffen ist. Wenn die Hybrid-RBS einmal aufgebaut ist unter Verwendung der EcoR1-Stelle zur Verbindung der $P_L$-SD-Sequenz mit der ATG-Startsequenz des Interferon kodierenden Gens, kann sie ferner durch Restriktion mit EcoR1, Auffüllen der Enden mit Klenow-Polymerase I und Verbinden der beiden anfallenden Enden durch stumpfendige Ligation mit $T_4$-DNA-Ligase modifiziert werden.

Die Erfindung wird durch die folgenden Beispiele weiter veranschaulicht.

**Beispiel 1**

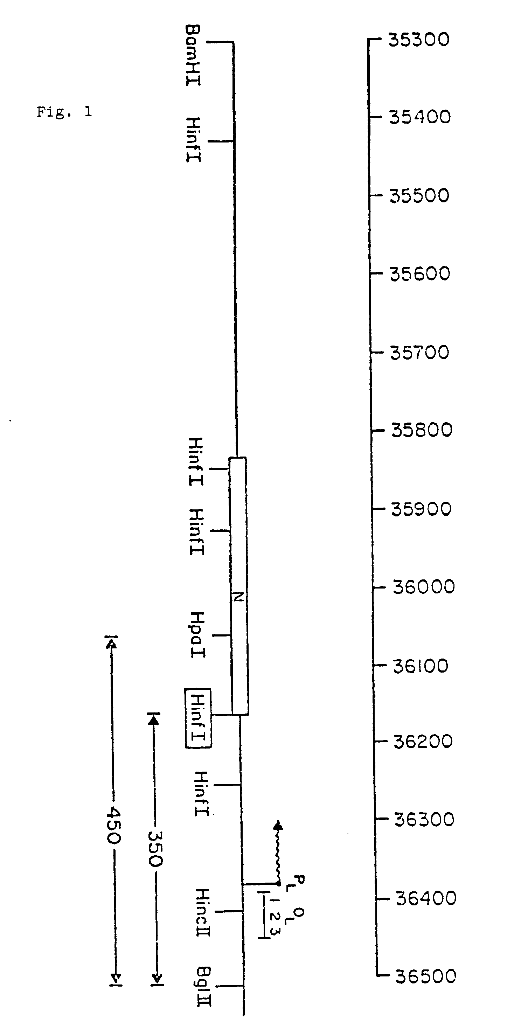

Um ein Fragment mit 350 Basenpaaren (BP), den $λP_L$-Promotor-$O_L$-Operator enthaltend, zu isolieren, wurden 250 µg λcl857 Sam7 DNA (Miles Laboratories) mit Restriktionsendonukleasen BamH1 und BglII verdaut und die Produkte wurden an Agarose-Gel durch Elektrophorese getrennt. Ein 1200 BP-Fragment mit dem $P_L$-Promotor-$O_L$-Operator wurde aus dem Gel isoliert (s. Fig. 1).

Dieses 1200 BP-Fragment wurde dann vollständig mit Hpal verdaut, was ein 450 BP-Fragment lieferte, das wieder mit Hinfl teilverdaut wurde. Ein 350 BP-Fragment wurde durch 5 % Polyacrylamid-Gel-Elektrophorese (PAGE) isoliert, das die $P_L$-Promotor- und $O_L$-Operator-Stellen ($O_{L1}$, $O_{L2}$, $O_{L3}$) enthält, an die sich der $\lambda$cl-Repressor bindet. Die Hinfl-Stelle, die zwischen der SD-Sequenz des $\lambda$N-Gens und dem initiierenden Kodon für das N-Gen auftritt (s. Fig. 1 und 2), ermöglicht den Aufbau von Hybrid-Ribosomenbindungsstellen für den Zweck der Expression fremder Gene in E. coli. Diese Hinfl-Stelle kann auch in eine EcoR1-Stelle umgewandelt werden.

Das 350 BP BglII - Hinfl-Fragment wurde in Plasmid pRC1 kloniert, das mit EcoR1 und BglII verdaut worden war. pRC1 ist ein Derivat von pBR322, das eine BglII-Stelle nahe der EcoR1-Stelle enthält (s. Fig. 2). Fig. 2 zeigt auch die Sequenzen der verbindenden Enden und gibt an, wie das EcoR1-Ende des Vektors sich an das Hinfl-Ende des Fragments binden kann und so die EcoR1-Stelle rekonstituiert (das eingekreiste A in Fig. 2 wurde durch Zellmechanismen in vivo wieder hergestellt). Das anfallende rekombinante Plasmid wurde mit pRC14 bezeichnet.

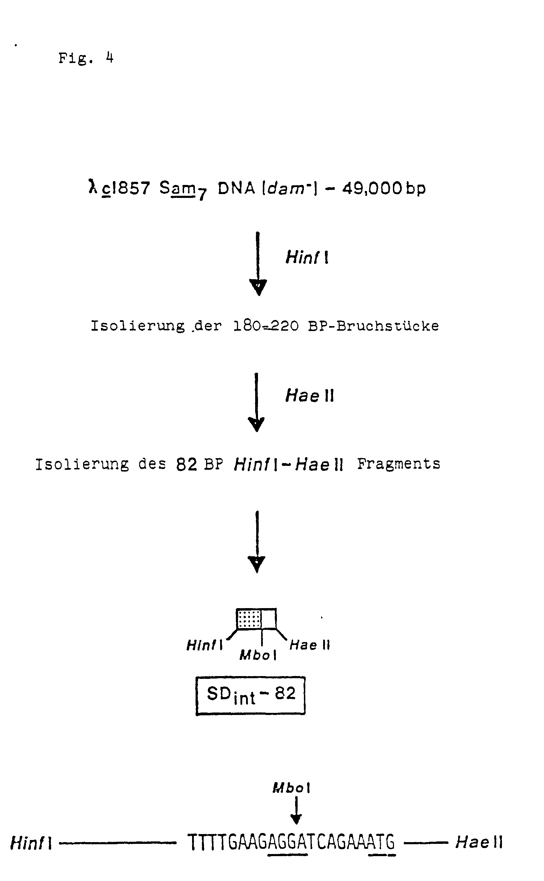

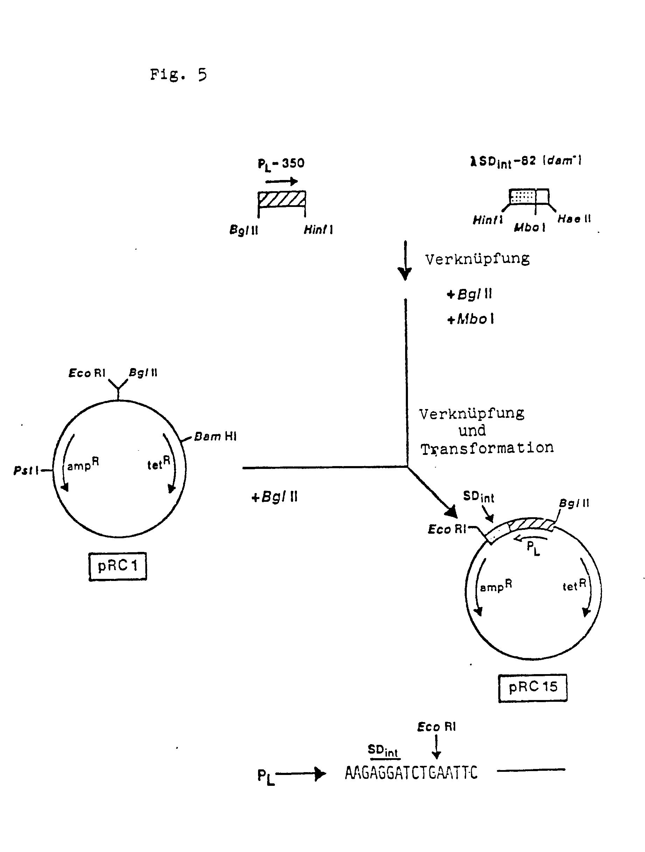

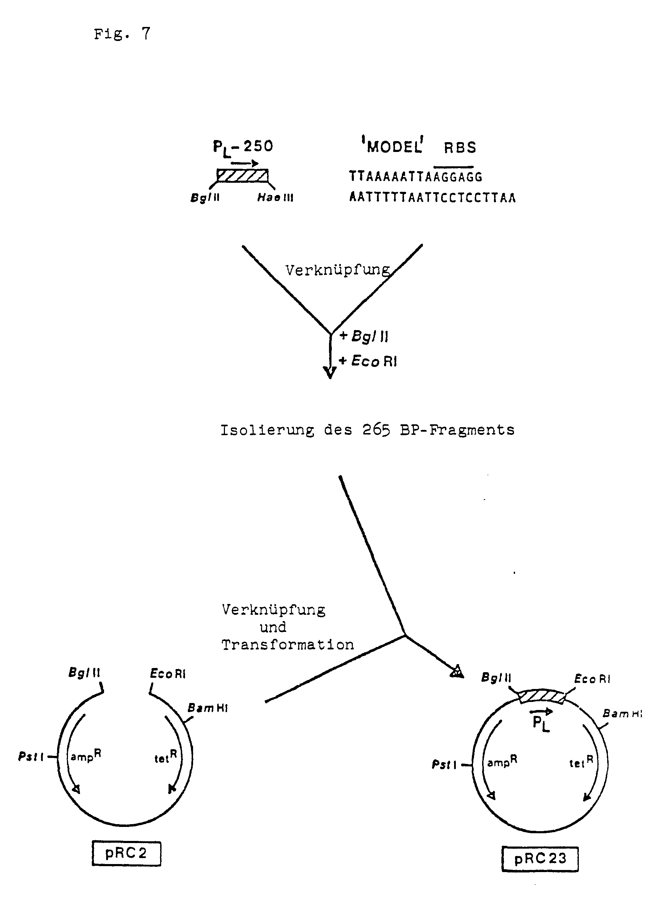

Eine Anzahl weiterer Expressionsvektoren wurden auch unter Verwendung des $P_L$-Promotors aufgebaut (Fig. 4, 5, 6 und 7). pRC15 enthält zusätzlich zu dem oben beschriebenen 350 BP BglII - Hinfl-Fragment ein 55 BP-Fragment (Hinfl - Mbol), das die SD-Sequenz des $\lambda$ int-Gens enthält (s. Fig. 4 und 5). pRC21 und pRC22 sind analog pRC14 bzw. pRC15. Der prinzipielle Unterschied zwischen diesen Plasmiden ist die Orientierung des eingesetzten $P_L$-haltigen Fragments und somit die Richtung der Transkription (s. Fig. 5). pRC23 wurde durch Binden synthetischer Oligonukleotide, eine "consensus"-RBS enthaltend (Scherer et al., Nucleic Acids Research 8, (1980) 3895), an ein 250 BP BglII - HaeIII-Fragment, den $P_L$-Promotor enthaltend, und Einfügen des Bindungsprodukts in pRC2, wie in Fig. 7 gezeigt, aufgebaut.

**Beispiel 2**

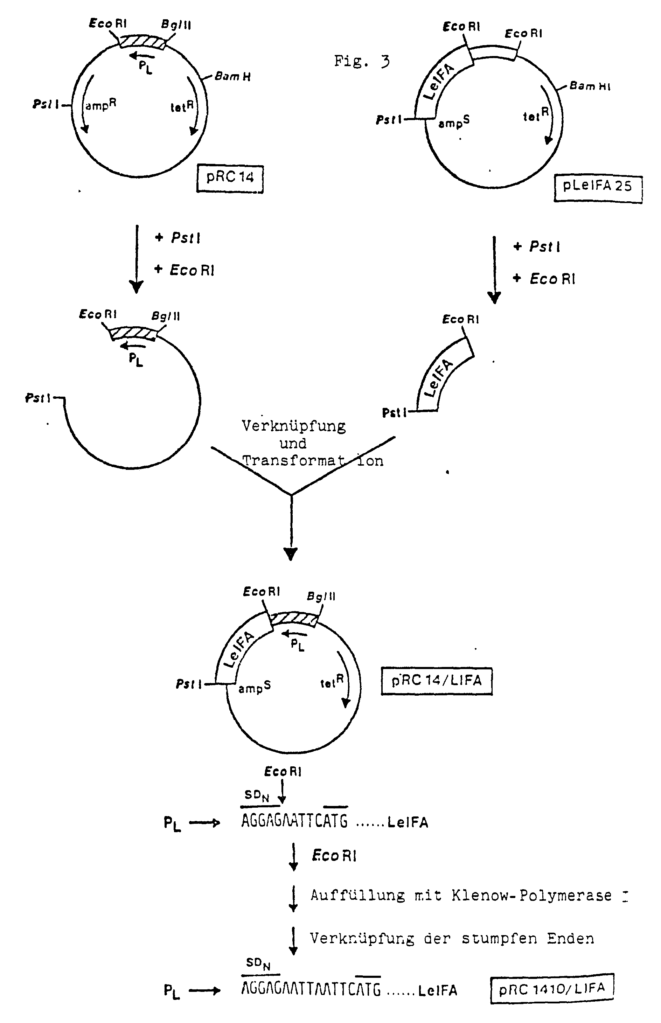

Das Leukozyteninterferon-A (nachfolgend hier zuweilen als LeIF-A oder LIFA bezeichnet)-Gen wurde in verschiedene Plasmide eingeführt und in Wirtsorganismen gemäß folgender Arbeitsweise exprimiert. Ein Pstl - EcoR1-Fragment aus pLeIF A25 (Goeddel et al., Nature 287, (1980) 411), der Quelle des LeIF-Gens, wurde isoliert und an das aus pRC14 isolierte große EcoR1 - Pstl-Fragment gebunden. Die Sequenz der Verbindung ist in Fig. 3 gezeigt. Die SD-Sequenz ist 5 Basen entfernt von dem ATG-Initiationskodon. Um diesen Abstand auf 9 zu erhöhen, wurde pRC14/LIFA mit EcoR1 verdaut, die kohäsiven Enden wurden mit dem Klenow-Fragment von Polymerase I (+dTTP, dATP) aufgefüllt und die stumpfen Enden erneut verknüpft. Die Sequenz der anfallenden Verbindung ist in Fig. 3 gezeigt (pRC1410/LIFA). Die Sequenz der neuen Hybrid-Ribosomenbindungsstellen, die in diesem Beispiel verwendet wurde, ist $\overline{A}\overline{G}\overline{G}\overline{A}\overline{G}$AATTC$\overline{A}\overline{T}$G für pRC14/LIFA und $\overline{A}\overline{G}\overline{G}\overline{A}\overline{G}$AATTAATTC$\overline{A}\overline{T}$G für pRC1410/LIFA (beide in Fig. 3 dargestellt).

Die vorgenannten $P_L$-Expressionsplasmide wurden in einen Stamm von E. coli (Stamm MA210) transformiert, der auf seinem Chromosom einen Defekt-Prophagen trägt. Das cl-Repressor-Gen des Prophagen enthält eine Mutation, clts857, die ihn temperaturempfindlich macht. Das heißt, der Repressor ist funktionell von etwa 30° C bis etwa 36°C und inaktiviert bei etwa 37°C bis etwa 42°C. Dieses Merkmal bietet einen bequemen Mechanismus zum Steuern des $P_L$-Promotors. In diesem Falle werden die zellen M9-Glucose-Medien bei 30° C bis auf 2 - 3x10^8 Zellen/ml vermehrt, dann 2 h auf 42°C gebracht. Die Zellen in der Kultur werden dann in 7M Guanidin-HCl lysiert und das Lysat getestet. Wenn diese Arbeitsweise gefolgt wird von pRC1410/LIFA, werden Ausbeuten von 10^7 bis 10^8 Einheiten/l Interferonaktivität in den Bakterienextrakten oder dem Lysat festgestellt (Tabelle 1). Der Grad an Expression in pRC14/LIFA ist etwa 10 % des in pRC1410/LIFA erhaltenen. pRC15/LIFA liefert Werte von LIFA-Expression, die vergleichbar sind mit pRC1410/LIFA (Tabelle 1).

**Tabelle 1**

| e. coli-Stamm | SD-AUG-Abstand (BP) | LeIF-A-Aktivität (E/ml)[d] | Anzahl der Versuche |
|---|---|---|---|
| MA210 (pRC14/LIFA)[a] | 6 | 320-1280 | 3 |
| MA210 (pRC1410/LIFA)[a] | 10 | 7680-15360 | 3 |
| MA210 (pRC15/LIFA)[a] | 9 | 15360 | 1 |
| 294 (pRC1410/LIFA, pRK248clts) | 10 | 10240 | 1 |
| 294 (pRC143/LIFA)[c] | 6 | 480 | 1 |
| 294 (pRC144/LIFA)[c] | 6 | 480 | 1 |

a) Das cl-Repressorgen im Stamm MA210 liegt als Defekt-Prophage am Wirtschromosom vor und enthält die temperaturempfindliche Mutation cl857.

b) Das cl-Repressorgen liegt als 2400 BP BglII-Insert in pRK248c-lts2 vor, einem wenig-kopierenden

Plasmid, das mit Derivaten von pBR322 kompatibel ist.

c) Das cl-Repressorgen liegt als 2400 BP-Insert an der BglII-Stelle von pRC14/LIFA in einer der beiden möglichen Orientierungen vor.

d) Die gezeigten Werte sind Daten, die aus Tests mit 1:50-Verdünnungen der Bakterienextrakte erhalten wurden. So werden 10 000 Einheiten/ml zu $5 \times 10^5$ Einheiten/ml Extrakt oder ungefähr $5 \times 10^4$ Einheiten/ml ($5 \times 10^7$ Einheiten/l) Kultur bei $5 \times 10^8$ Zellen/ml.

## Beispiel 3

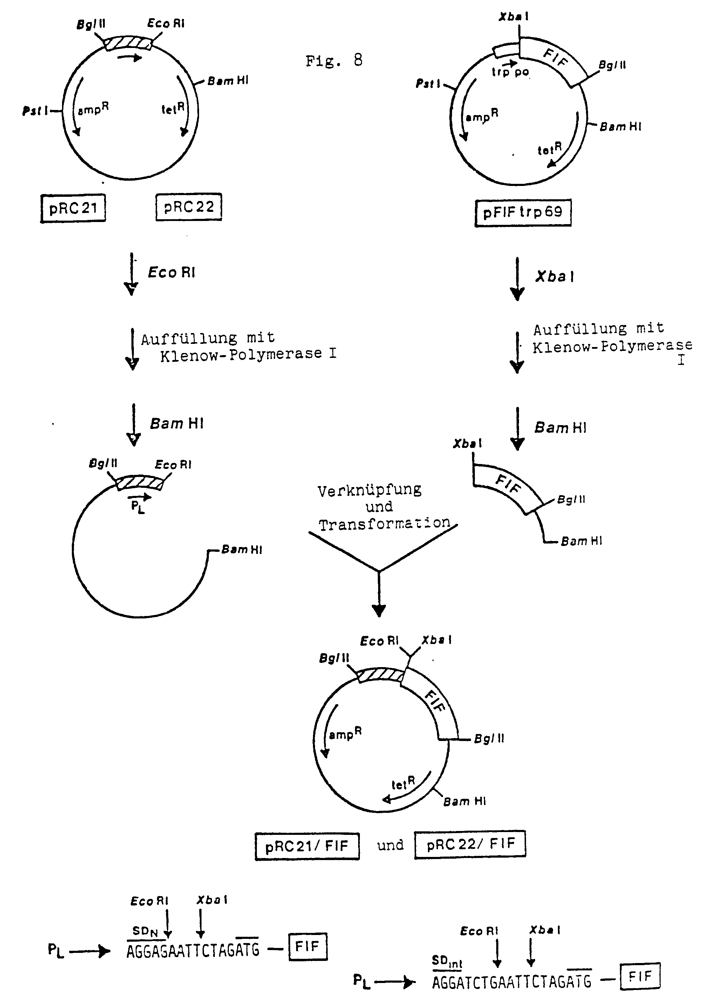

Das Human-Fibroblasten-Interferon-Gen (FIF) wurde in die Vektoren pRC21 und pRC22 wie folgt eingesetzt (Fig. 8). In getrennten Reaktionen wurden pRC21 und pRC22 mit EcoR1 verdaut, die Enden mit Klenow-Polymerase I aufgefüllt, mit BamHI verdaut und das große Fragment isoliert (4,3 Kb). pFIF trp 69 (Goeddel et al., Nucleic Acids Res. 8, (1980) 4057), die Quelle des FIF-Gens, wurde mit XbaI verdaut, die Enden in stumpfe Enden durch Auffüllen mit Klenow-Polymerase I umgewandelt, mit BamHI verdaut und das kleinere Fragment (850 BP), das FIF-Gen enthaltend, wurde isoliert. Das FIF 850 BP-Fragment wurde an das 4,3 Kb $P_L$-enthaltende Fragment gebunden und die erhaltenen Produkte durch Restriktionsanalyse bestätigt.

Um die Fähigkeit der $P_L$-FIF-Plasmide zur Expression von FIF zu testen, wurden mit pRC21/FIF und pRC22/FIF transformierte E. coli-Zellen bei 30°C in M9-Glucosemedien bis zu einer Zelldichte von 2 - $3 \times 10^8$ Zellen/ml gezüchtet und bei 42°C induziert. 1 ml-Proben wurden genommen, Zellen durch Zentrifugieren gesammelt und wieder in 7M Guanidin-HCl ($5 \times 10^9$ Zellen/ml) suspendiert, um die Zellen zu lysieren. Zelltrümmer wurden abzentrifugiert, und die überstehende Flüssigkeit wurde vor dem Test auf antivirale Aktivität 50-fach verdünnt. Die Ergebnisse sind in Tabelle 2 dargestellt.

## Tabelle 2

| E. coli-Stamm | Inkubationstemp. (°C)-Zeit | FIF-Aktivität (E/ml Extrakt) |
|---|---|---|
| RR1(pRK248clts, pRC21/FIF) | 30° - Kontrolle | 0 |
| '' | 42° - 0.5 h | 1024 |
| '' | 42° - 1 h | 2048 |
| '' | 42° - 2 h | 4096 |
| '' | 42° - 3 h | 256 |
| RR1(pRK248clts, pRC22/FIF) | 30° - Kontrolle | 128 |
| '' | 42° - 0.5 h | 484 |
| '' | 42° - 1 h | 256 |
| '' | 42° - 2 h | 2048 |
| '' | 42° - 3 h | 512 |

## Beispiel 4

Zur Bestimmung des Einflusses neuer hybrider Ribosomenbindungsstellen auf die FIF-Expression wurden Derivate von pRC21/FIF und pRC22/FIF aufgebaut. Die Kombination der beiden Restriktionsstellen im Verknüpferbereich von pRC21/FIF und pRC22/FIF (s. Fig. 8) bot ein Mittel zum Einführen verschiedener Modifikationen in die Hybrid-RBS. EcoR1 oder XbaI wurden zum Schneiden innerhalb des Verknüpferbereichs verwendet, Klenow-Polymerase I wurde zum Auffüllen der anfallenden Enden verwendet und die stumpfen Enden wurden erneut verknüpft, wodurch die verschiedenen in Tabelle 3 gezeigten Sequenzen entstanden. Diese Produkte wurden auf FIF-Expression in einem transformierten E. coli-Stamm RR1-(pRK248clts) getestet. Um die Fähigkeit dieser $P_L$-FIF-Plasmide zur FIF-Expression zu testen, wurde in der nachfolgend beschriebenen Weise für jede Kultur von Zellen vorgegangen, die mit einer der folgenden Plasmid-Art transformiert waren: pRC21/FIF, pRC22/FIF, pRC211/FIF, pRC221/FIF, pRC212/FIF und pRC222/FIF.

Die Zellen wurden bei 30°C in M9-Glucosemedien zu einer Zelldichte von 2 - $3 \times 10^8$ Zellen/ml gezüchtet und bei 42°C für etwa 120 min induziert, 1 ml-Proben wurden genommen, Zellen durch Zentrifugieren gesammelt und wieder in 7M Guanidin-HCl ($5 \times 10^9$ Zellen/ml) suspendiert, um die Zellen zu lysieren. Zelltrümmer wurden abzentrifugiert und die überstehende Flüssigkeit vor dem Test auf antivirale Aktivität 50-fach verdünnt. Die Ergebnisse sind in Tabelle 3 gezeigt.

**Tabelle 3**

| Plasmid | Sequenz der neuen Hybrid-RBS | S̄D̄ - ĀT̄Ḡ Abstand | FIF-Aktivität E/ml |
|---|---|---|---|
| pRC21/FIF | ĀḠḠĀḠAATTCTAGĀT̄Ḡ | 8 | 10240 |
| pRC22/FIF | ĀḠḠĀTCTGAATTCTAGĀT̄Ḡ | 12 | 2560 |
| pRC211/FIF | ĀḠḠĀḠAATTAATTCTAGĀT̄Ḡ | 12 | 5120 |
| pRC221/FIF | ĀḠḠĀTCTGAATTAATTCTAGĀT̄Ḡ | 16 | 320 |
| pRC212/FIF | ĀḠḠĀḠAATTCTAGCTAGĀT̄Ḡ | 12 | 160 |
| pRC222/FIF | ĀḠḠĀTCTGAATTCTAGCTAGĀT̄Ḡ | 16 | 320 |

**Beispiel 5**

Es wurden auch P$_L$-Expressionsvektoren zusammengesetzt, die das Human-Immun-Interferon (IFI)-Gen enthielten. Die Quelle für das IFI-Gen war pHIT3709, ein Derivat von pBR322 mit einer 1100 BP cDNA-Kopie von IFI-mRNA, eingesetzt an der PstI-Stelle. Die kodierende Sequenz für IFI, auf diesem Insert vorhanden, ist durch die Formel I repräsentiert (s. Seite 7). Das Plasmid pHIT 3709 mit dem IFI-Gen wurde nach folgender Arbeitsweise aufgebaut:

(i) Isolieren von mRNA, die für IFI kodiert

Aus peripherem menschlichem Blut präparierte Lymphozyten wurden in RPMI-1640-Medium (10 % totales Rinderserum enthaltend) mit einem Gehalt von 15ng/ml 12-O-Tetradecanoylphorbol-13-acetat (TPA) und 40 µg/ml Concanavalin A, bei 37°C zur IFI-Induktion inkubiert. Nach 24-stündiger Inkubation wurden die so induzierten Human-Lymphozyten (1x10$^{10}$ Zellen) zerstört und denaturiert in einer Thioguanidinlösung (5 M Guanidinthiocyanat, 5 % Mercaptoethanol, 50 mM Tris-HCl (pH 7,6), 10 mM EDTA) in einem Teflonhomogenisator. Dann wurde Natrium-N-lauroyl-sarcosinat in einer Konzentration von 4 % zugesetzt und das Gemisch nach dem Homogenisieren über 6 ml Caesiumchlorid-Lösung (5,7 M Caesiumchlorid, 0,1 M EDTA) geschichtet und bei 15°C und 24 000 UpM 30 h unter Einsatz eines Beckman SW27-Rotors zu einem RNA-Niederschlag zentrifugiert.

Dieser RNA-Niederschlag wurde in 0,25 % N-Lauroylsarcosinat gelöst und dann mit Ethanol ausgefällt, um 8,3 mg RNA zu ergeben. Diese RNA konnte in hochkonzentrierter Salzlösung (0,5 M NaCl, 10 mM Tris-HCl (pH 7,6), 1 mM EDTA, 0,3 % SDS) an einer Oligo-(dT) Zellulose-Säule absorbiert werden, und die poly(A)-haltige mRNA wurde mit einer Salzlösung geringer Konzentration (10 mM Tris-HCl (pH 7,6 ), 1 mM EDTA, 0,3 % SDS) eluiert. Es wurden 700 µg mRNA gesammelt.

Diese mRNA wurde wieder mit Ethanol ausgefällt, dann in 0,2 ml einer Lösung von 10 mM Tris-HCl (pH 7,6), 2 mM EDTA und 0,3 % SDS gelöst, 2 min bei 65°C behandelt und in 22 Portionen durch 10 - 35 % Saccharose-Dichtegradienten-Zentrifugation bei 20°C und 25 000 UpM für 21 h mit einem Beckman SW 27-Rotor fraktioniert. Teilmengen einer jeden Fraktion wurden in Xenopus laevis-Oozyten injiziert, und die synthetisierten Proteine wurden auf Interferonaktivität getestet. So zeigte sich, daß Fraktion 12 (mit einem Sedimentationskoeffizienten von 12 - 14S) eine Aktivität von 195 internationalen Interferoneinheiten pro Mikrogramm mRNA hatte. Die mRNA in der so erhaltenen Fraktion 12 wog etwa 20 µg.

(ii) Synthese von einsträngiger DNA

100 µl dines Reaktionsgemischs, das 5 µg der obigen mRNA, 50 µg Oligo(dT), 100 Einheiten reverser Transkriptase, je 1 mM dATP, dCTP, dGTP und dTTP, 8 mM MgCl$_2$, 50 mM KCl, 10 mM Dithiothreitol und 50 mM Tris-HCl enthielt (pH 8,3), wurden 1 h bei 42°C inkubiert, dann mit Phenol entproteinisiert und mit 0,1 n NaOH bei 70°C 20 min zum Abbau von RNA behandelt.

(iii) Synthese von doppelsträngiger DNA

Die so synthetisierte einsträngige komplementäre DNA wurde in 50 µl eines Reaktionsgemischs mit 100 Einheiten reverser Transkriptase, je 1 mM dATP, dCTP, dGTP und dTTP, 8 mM MgCl$_2$, 50 mM KCl, 10 mM Dithiothreitol und 50 mM Tris-HCl (pH 8,3) der Reaktion bei 42°C für 2 h zur Synthese der doppelsträngigen (ds) DNA unterzogen.

(iv) Addition von dC-Schwänzen

Die obige doppelsträngige DNA wurde mit 60 Einheiten Nuklease S-1 in 50 µl eines Reaktionsgemischs behandelt, das 0,1 M Natriumacetat (pH 4,5), 0,25 M NaCl, 1,5 mM ZnSO$_4$ enthielt, und zwar 30 min bei Raumtemperatur. Das Reaktionsgemisch wurde mit Phenol entproteinisiert, die DNA wurde mit Ethanol ausgefällt und terminaler Transferasereaktion in einem Gemisch mit 0,14 M Kalium-Kakodylat, 0,3 M Tris (pH 7,6), 2 mM Dithiothreitol, 1 mM $CoCl_2$, 0,15 mM dCTP und 30 Einheiten terminaler Transferase bei 37°C für 3 min zwecks Addition von etwa 20 Desoxycytidin-Resten an jedem 3'-Ende der dsDNA unterzogen.

Diese Reaktionsfolge lieferte etwa 300 ng einer doppelsträngigen, Desoxycytidinschwänze-enthaltenden DNA.

### (v) Spaltung von E. coli-Plasmid pBR322 und Addition von dG- Schwänzen

Separat wurden 10 µg E. coli-Plasmid pBR322-DNA mit 20 Einheiten Restriktionsenzym PstI in 50 µl eines Reaktionsgemischs mit 50 mM NaCl, 6 mM Tris-HCl (pH 7,4), 6 mM $MgCl_2$, 6 mM 2-Mercaptoethanol und 100 µg/ml Rinderserum-Albumin bei 37°C 3 h behandelt. Das Reaktionsgemisch wurde dann mit Phenol entproteinisiert,, und die DNA wurde ferner mit 30 Einheiten terminaler Transferase in 50 µl eines Reaktionsgemischs mit 0,14 M Kaliumkakodylat, 0,3 M Tris (pH 7,6), 2 mM Dithiothreitol, 1 mM $CoCl_2$ und 0,15 mM dGTP bei 37°C 3 min zwecks Addition von etwa 8 Desoxyguanidin-Resten an jedem 3'-Ende der Plasmid-pBR322-DNA behandelt.

### (vi) Verschmelzen von cDNA und Transformation von E. coli

Das Verschmelzen erfolgte durch Erhitzen von 0,1 µg der so erhaltenen dC-schwänzigen synthetischen dsDNA und 0,5 µg der obigen dG-schwänzigen Plasmid-pBR322-DNA in einer Lösung mit 0,1 M NaCl, 50 mM Tris-HCl (pH 7,6) und 1 mM EDTA bei 65°C für 2 min und dann bei 45°C für 2 h, gefolgt von allmählichem Abkühlen. Die Transformation von E. coli X1776 erfolgte nach der Methode von Enea et al. (J. Mol. Biol. 96, (1975) 495).

### (vii) Isolierung von cDNA-haltigem Plasmid

Etwa 8500 Tetracyclin-resistente Kolonien wurden so isoliert, und die DNA einer jeden Kolonie wurde an ein Nitrocellulosefilter fixiert (M. Grunstein und D.S. Hogness, Proc. Natl. Acad. Sci. USA, 72, (1975) 3961).

Separat wurden, basierend auf der Aminosäuresequenz von IFI, wie von Gray et al. (Nature 295, (1982) 503) berichtet, zwei Basensequenzen 5' TCCTGGCA$^A_G$TA$^A_G$C 3' und 5' AC$^G_A$TTCAT$^G_A$TCT$^T_C$TCT$^T_C$T 3' entsprechend Aminosäuresequenzen Cys-Tyr-Cys-Gln-Asp (1 - 5) und Lys-Gln-Asp-Met-Asn-Val (77 - 82) chemisch nach der Triestermethode synthetisiert (R. Crea et al., Proc. Natl. Acad. Sci. USA, 75, (1978) 5765). 0,2 µg dieser Oligonucleotide wurden mit 3 Einheiten T4-Polynukleotidkinase in 50 µl eines Reaktionsgemischs mit 50 mM Tris-HCl (pH 8,0), 10 mM $MgCl_2$, 10 mM Mercaptoethanol und 50 µCi γ-$^{32}$P-ATP bei 37°C 1 h behandelt. Diese am 5'-Ende mit $^{32}$P markierten Oligonukleotide wurden als Sonden verwendet und mit der DNA auf dem oben genannten Nitrocellulosefilter nach der Methode von Lawn et al. (Nucleic Acids Res. 9, (1981) 6103) angelagert. Durch Autoradiographie wurden 4 gegenüber den obigen beiden Oligonukleotidsonden reaktive Kolonien identifiziert.

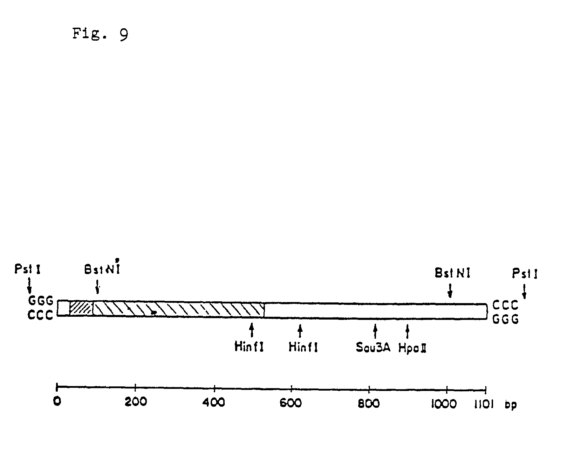

Plasmid-DNAs wurden aus den Bakterienzellen einer jeden dieser Kolonien nach der Methode von Birnboim und Doly (Nucleic Acids Res. 1, (1979) 1513) isoliert. Die Inserts in den Plasmid-DNAs wurden mit PstI-Restriktionsenzym herausgeschnitten. Von den isolierten Plasmiden wurde das jenige mit dem längsten cDNA-Insert ausgewählt und "pHIT3709" genannt. Die Restriktionsenzymkarte dieses Plasmids ist in Fig. 9 gezeigt.

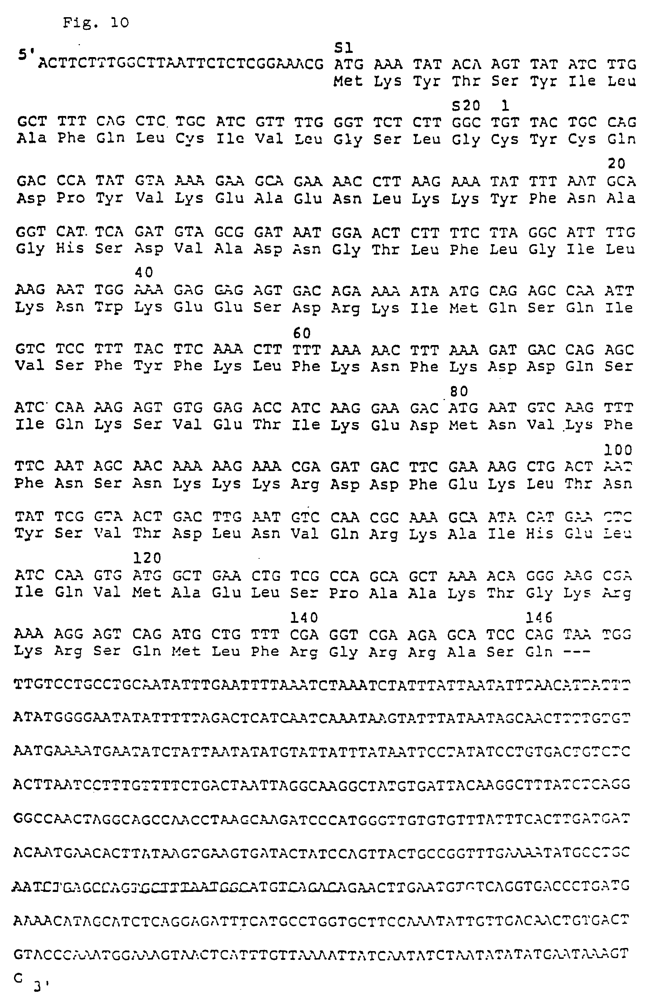

Die Primärstruktur (Basensequenz) der in das pHIT3709-Plasmid eingesetzten mRNA-Sequenz wurde dann nach der Methode von Sanger et al. (Proc. Natl. Acad. Sci. USA 74, (1977) 5463) und nach der Maxam-Gilbert-Methode bestimmt. Die Primärstruktur ist in Fig. 10 dargestellt.

Diese Primärstruktur steht im Einklang mit der von Gray et al. für IFI cDNA angegebenen, mit der Ausnahme, dass die Aminosäureposition 140 durch CGA (Arg) anstatt durch CAA (Gln) kodiert ist.

Aus der Primärstruktur ist klar, daß dieses Plasmid den gesamten kodierenden Bereich für das IFI-Protein enthält. Diese Tatsache deutet die Möglichkeit an, Wirte, wie E. coli, Immuninterferon produzieren zu lassen, indem die in dieses Plasmid eingesetzte DNA-Sequenz auf ein anderes Expressionsplasmid überführt wird.

**Beispiel 6**

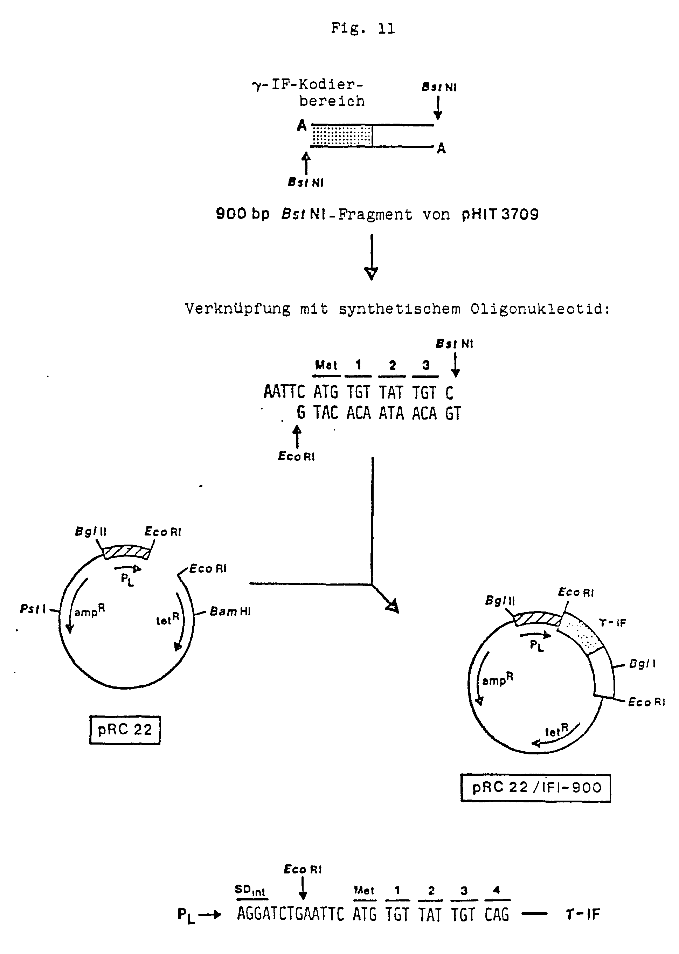

Ein 900 BP BstN1-Fragment wurde aus pHIT3709 isoliert, das 430 BP der kodierenden Sequenz für IFI und 470 BP des 3'-nichtkodierenden Bereichs enthält. Nicht vorhanden in diesem Fragment sind die Sequenzen für das "Signal"-Protein von IFI und die Kodons für die ersten drei Aminosäuren des reifen Proteins. Um die drei fehlenden Kodons wieder herzustellen und ein Met-Startkodon zu schaffen, wurden synthetische Oligonukleotide hergestellt und mit dem 900 BP-Fragment verknüpft (s. Fig. 11). Das synthetische Segment wandelte beide BstN1-Enden in EcoR1-Enden um. Das anfallende Fragment wurde in den Vektor pRC22 kloniert, das der Restriktion mit EcoR1 unterzogen worden war. Die Orientierung des Inserts wurde durch Restriktionsanalyse mit BglI bestimmt, das innerhalb des 3'-nichtkodierenden Bereichs schneidet.

Der pRC22-Vektor mit dem IFI-Gen (pRC22/IFI-900) wurde über die Promotor-Gen-Verbindung sequenziert, um sicherzustellen, daß alle Verknüpfungen wie erwartet eingetreten waren (s. Fig. 11).

9

Zum Test auf die Expression des IFI-Gens wurde E. coli, Stamm RRI (pRK248clts, pRC22/IFI-900) in M9-Glucosemedien bei 30°C zu 3 - 4x10$^8$ Zellen/ml gezüchtet, dann eine Stunde bei 42°C induziert. Vor der Induktion wurden zusätzliche Glucose und Casaminosäuren zu 1,0 % bzw. 0,5 % zugesetzt. Eine 10-ml-Probe wurde genommen, die Zellen wurden durch Zentrifugieren gesammelt und erneut in 0,1 ml 50 mM Tris (pH 7,4), 10 % Saccharose, suspendiert und die Suspension in einem Trockeneis/Ethanol-Bad schnellgefroren. Die Zellen wurden bei 20°C aufgetaut, dann in ein Eisbad übergeführt. NaCl wurde zu 100 mM zugesetzt, EDTA zu 10 mM, Spermidin zu 20 mM und Lysozym zu 200 µg/ml. Das Gemisch wurde 45 min auf Eis gehalten, dann 2 min bei 37°C inkubiert. Zelltrümmer wurden durch Zentrifugieren entfernt und die überstehende Flüssigkeit auf IFI-antivirale Aktivität an WISH-Zellen getestet. Dieses erste Experiment führte zu einer Ausbeute von 1280 Einheiten IFI-Aktivität pro ml Zellextrakt.

Zur Bestimmung der Kinetik der Induktion wurde die obige Arbeitsweise wiederholt. Eine Probe wurde als Kontrolle bei 30°C gehalten und weitere Proben wurden 30, 60, 90, 120 und 180 min nach Induktion bei 42°C genommen. Die Proben wurden wie oben beschrieben bearbeitet. Die in Tabelle 4 dargestellten Ergebnisse zeigen, dass bei 30°C keine Aktivität vorliegt und dass etwa 90 min nach Induktion bei 42°C die nachgewiesene Aktivität ein Maximum erreichte und dann allmählich abfiel.

**Tabelle 4**

| E. coli-Stamm | Induktionsbedingungen Temp. (°C)/Zeit | IFI-Aktivität |
|---|---|---|
| RR1 (pRK248clts, pRC22/IFI-900) | 30° | 0 |
| '' | 42°/30 min | 120 |
| '' | 42°/60 min | 120 |
| '' | 42°/90 min | 320 |
| '' | 42°/120 min | 160 |
| '' | 42°/180 min | 40 |

**Beispiel 7**

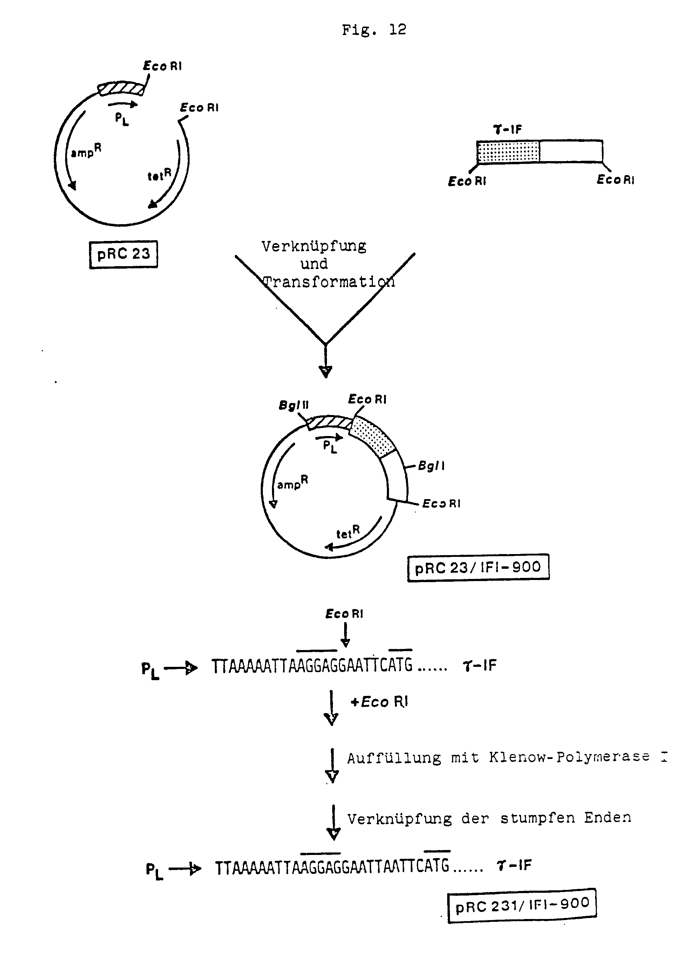

pRC22/IFI-900-DNA wurde weiter der Restriktion mit EcoR1 unterworfen, und das 900 BP-Fragment mit dem IFI wurde isoliert und in pRC23 eingesetzt, das der Restriktion mit EcoR1 unterworfen worden war (s. Fig. 12). Das resultierende Produkt, pRC23/IFI-900, enthielt eine RBS, die von der in pRC22/IFI beträchtlich verschieden war (vgl. Fig. 11 & 12). Zum Test auf Expression des IFI-Gens wurde der Stamm RRI(pRK248clts, pRC23/IFI-900) in M9-Glucosemedien bei 30°C zu 3 - 4x10$^8$ Zellen/ml gezüchtet, dann eine Stunde bei 42°C induziert. Vor der Induktion wurden weitere Glucose und Casaminosäuren zu 1,0 bzw. 0,5 % zugesetzt. Eine 10 ml-Probe wurde genommen, die Zellen Wurden durch Zentrifugieren gesammelt und erneut in 0,1 ml 50 mM Tris (pH 7,4), 10 % Saccharose, suspendiert und die Suspension in einem Trockeneis/Ethanol-Bad schnellgefroren. Die Zellen wurden bei 20°C aufgetaut, dann in ein Eisbad übergeführt. NaCl wurde zu 100 mM zugesetzt, EDTA zu 10 mM, Spermidin zu 20 mM und Lysozym zu 200 µg/ml. Das Gemisch wurde 45 min auf Eis gehalten, dann 2 min bei 37°C inkubiert. Zelltrümmer wurden abzentrifugiert, und die überstehende Flüssigkeit wurde auf IFI-antivirale Aktivität an WISH-Zellen getestet. pRC23/IFI ergab nach Transformation von E. coli, Stamm RRI, etwa viermal mehr IFI-Aktivität als pRC22/IFI, wie Tabelle 5 zeigt:

**Tabelle 5**

| E. coli-Stamm | Induktionsbedingungen (°C/min) | IFI-Aktivität (E/ml) |
|---|---|---|
| RR1 (pRK248clts, pRC22/IFI-900) | 42°/90 | 320 |
| RR1 (pRK248clts, pRC23/IFI-900) | 42°/90 | 1280 |
| RR1 (pRK248clts, pRC231/IFI-900) | 42°/90 | 640 |

**Beispiel 8**

pRC23/IFI wurde weiter der Restriktion mit EcoR1 unter Bedingungen unterworfen, die dazu führten, daß nur eine der beiden Stellen geschnitten wurde. Die anfallenden Moleküle wurden dann mit Klenow-Polymerase I zum Auffüllen der EcoR1-Enden behandelt. Die stumpfen Enden wurden mit T₄-DNA-Ligase miteinander verknüpft und die DNA zum Transformieren von RRI(pRK248clts) verwendet. Die Transformanten wurden auf Verlust der EcoR1-Stelle am Beginn des IFI-Gens geprüft, und zwei wurden gefunden. Eines von ihnen, pRC231/IFI-900, wurde zur Transformation von E. coli, Stamm RR1, zwecks Expression von IFI verwendet. Zum Test auf Expression des IFI-Gens wurde dieser Stamm RRI(pRK248-clts, pRC231/IFI-900) in M9-Glucosemedium bei 30°C zu 3 - 4x10⁸ Zellen/ml gezüchtet, dann eine Stunde bei 42°C induziert. Vor der Induktion wurden zusätzliche Glucose und Casaminosäuren zu 1,0 bzw. 0,5 % zugesetzt. Eine 10-ml-Probe wurde genommen, die Zellen wurden durch Zentrifugieren gesammelt und erneut in 0,1 ml 50 mM Tris (pH 7,4), 10 % Saccharose, suspendiert und die Suspension in einem Trockeneis/Ethanol-Bad schnellgefroren. Die Zellen wurden bei 20°C aufgetaut, dann in ein Eisbad übergeführt. NaCl wurde zu 100 mM zugesetzt, EDTA zu 10 mM, Spermidin zu 20 mM und Lysozym zu 200 µg/ml. Das Gemisch wurde 45 min auf Eis gehalten, dann bei 37°C 2 min inkubiert. Zelltrümmer wurden abzentrifugiert und die überstehende Flüssigkeit auf IFI-antivirale Aktivität an WISH-Zellen getestet. Die Ergebnisse sind in Tabelle 5 gezeigt.

Die beschriebene Modifikation von pRC23/IFI war so ausgelegt, dass der Verknüpfungsbereich von 6 auf 10 BP verlängert wurde. Die gleiche Modifikation beim LeIF-A-Expressionsvektor brachte eine 10- bis 20-fache Zunahme der Expression. Im Falle der Expression von IFI scheint ein Abstand von 6 BP besser zu sein als von 10 BP.

**Patentansprüche**

1. Zur Expression von reifem Human-Interferon fähiger Vektor enthaltend die vom Bakteriophagen λ stammende P_L Promoter- und O_L Operator-DNA-Sequenz, eine reifes Human-Interferon kodierende DNA-Sequenz und eine eine hybride Ribosomenbindungsstelle kodierende DNA-Sequenz, die die besagte P_L Promoter- und O_L Operator-DNA-Sequenz stromabwärts und die besagte Human-Interferon kodierende DNA-Sequenz stromaufwärts verbindet und die durch eine der folgenden Sequenzen definiert ist:

AGGAGAATTCATG:
AGGAGAATTAATTCATG;
AGGAGAATTCTAGATG:
AGGATCTGAATTCTAGATG:
AGGAGAATTAATTCTAGATG:
AGGATCTGAATTAATTCTAGATG;
AGGAGAATTCTAGCTAGATG:
AGGATCTGAATTCTAGCTAGATG:
TTAAAAATTAAGGAGGAATTCATG und
TTAAAAATTAAGGAGGAATTAATTCATG.

2. Expressionsvektor nach Anspruch 1, dessen reifes Human-Interferon kodierende DNA-Sequenz reifes Human-Immun-Interferon kodiert.

3. Expressionsvektor nach Anspruch 2, dessen reifes Human-Immun-Interferon kodierende DNA-Sequenz durch die allgemeine Formel

(5') TGT TAC TGC CAG GAC CCA TAT GTA AAA GAA
GCA GAA AAC CTT AAG AAA TAT TTT AAT GCA GGT
CAT TCA GAT GTA GCG GAT AAT GGA ACT CTT TTC
TTA GGC ATT TTG AAG AAT TGG AAA GAG GAG AGT
GAC AGA AAA ATA ATG CAG AGC CAA ATT GTC TCC
TTT TAC TTC AAA CTT TTT AAA AAC TTT AAA GAT
GAC CAG AGC ATC CAA AAG AGT GTG GAG ACC ATC
AAG GAA GAC ATG AAT GTC AAG TTT TTC AAT AGC
AAC AAA AAG AAA CGA GAT GAC TTC GAA AAG CTG
ACT AAT TAT TCG GTA ACT GAC TTG AAT GTC CAA
CGC AAA GCA ATA CAT GAA CTC ATC CAA GTG ATG
GCT GAA CTG TCG CCA GCA GCT AAA ACA GGG AAG
CGA AAA AGG AGT CAG ATG CTG TTT CGA GGT CGA
AGA GCA TCC CAG -X (3')                                      (I),

worin X TAA, TGA oder TAG ist, dargestellt wird.

4. Expressionsvektor nach Anspruch 2, dessen reifes Human-Immun-Interferon Kodierende DNA-Sequenz ein Polypeptid der Formel

H₂N- Cys-Tyr-Cys-Gln-Asp-Pro-Tyr-Val-Lys-Glu-Ala-Glu-
Asn-Leu-Lys-Lys-Tyr-Phe-Asn-Ala-Gly-His-Ser-Asp-
Val-Ala-Asp-Asn-Gly-Thr-Lei-Phe-Leu-Gly-Ile-Leu-
Lys-Asn-Trp-Lys-Glu-Glu-Ser-Asp-Arg-Lys-Ile-Met-
Gln-Ser-Gln-Ile-Val-Ser-Phe-Tyr-Phe-Lys-Leu-Phe-
Lys-Asn-Phe-Lys-Asp-Asp-Gln-Ser-Ile-Gln-Lys-Ser-
Val-Glu-Thr-Ile-Lys-Glu-Asp-Met-Asn-Val-Lys-Phe-
Phe-Asn-Ser-Asn-Lys-Lys-Lys-Arg-Asp-Asp-Phe-Glu-
Lys-Leu-Thr-Asn-Tyr-Ser-Val-Thr-Asp-Lei-Asn-Val-
Gln-Arg-Lys-Ala-Ile-His-Glu-Leu-Ile-Gln-Val-Met-
Ala-Glu-Leu-Ser-Pro-Ala-Ala-Lys-Thr-Gly-Lys-Arg-
Lys-Arg-Ser-Gln-Met-Leu-Phe-Arg-Gly-Arg-Arg-Ala-
Ser-Gln-OH

kodiert.

5. Expressionsvektor nach einem der Ansprüche 2 - 4, dessen die hybride Ribosomenbindungsstelle kodierende DNA-Sequenz durch die Formel

TTAAAAATTAAGGAGGAATTCATG

dargestellt wird.

6. Expressionsvektor nach einem der Ansprüche 2 bis 4, dessen die hybride Ribosomenbindungsstelle kodierende DNA-Sequenz durch die Formel

TTAAAAATTAAGGAGGAATTAATTCATG

dargestellt wird.

7. Expressionsvektor nach Anspruch 1, dessen reifes Human-Interferon kodierende DNA-Sequenz reifes Human-Leukozyten-Interferon kodiert.

8. Expressionsvektor nach Anspruch 7, dessen die hybride Ribosomenbindungsstelle kodierende DNA-Sequenz durch die Formel

AGGAGAATTCATG oder AGGAGAATTAATTCATG

dargestellt wird.

9. Expressionsvektor nach Anspruch 1, dessen reifes Human-Interferon kodierende DNA-Sequenz reifes Human-Fibroblasten-Interferon kodiert.

10. Expressionsvektor nach Anspruch 9, dessen die hybride Ribosomenbindungsstelle kodierende DNA-Sequenz durch eine der folgenden Sequenzen definiert ist:

AGGAGAATTCTAGATG:
AGGATCTGAATTCTAGATG:
AGGAGAATTAATTCTAGATG:
AGGATCTGAATTAATTCTAGATG:
AGGAGAATTCTAGCTAGATG und
AGGATCTGAATTCTAGCTAGATG.

11. Ein Bakterium transformiert mit einem Expressionsvektor gemäss einem der Ansprüche 1 bis 10.

12. Ein Bakterium gemäss Anspruch 11, das Escherichia coli ist.

13. Verfahren zur Herstellung von reifem Human-Interferon, dadurch gekennzeichnet, dass man

(a) ein Bakterium mit einem Expressionsvektor gemäss einem der Ansprüche 1 bis 10 transformiert:

(b) das transformierte Bakterium züchtet, wodurch dieses das durch das Interferongen eines Expressionsvektors der Ansprüche 1 bis 10 kodierte reife Human-Interferon produziert;

(c) das transformierte Bakterium lysiert und

(d) das besagte Interferon aus dem anfallenden Lysat isoliert.

14. Verfahren nach Anspruch 13, dadurch gekennzeichnet, dass das Bakterium auf seinem Chromosom ein vom λ-Bakteriophagen stammendes mutantes cl-Repressorgen enthält, das für ein temperaturempfindliches Repressorprotein kodiert.

15. Verfahren nach Anspruch 13, bei dem der in Stufe a) verwendete Expressionsvektor ein vom λ-Bakteriophagen stammendes mutantes cl-Repressorgen enthält, das ein temperaturempfindliches Repressorprotein kodiert.

16. Verfahren nach Anspruch 13, bei dem das Bakterium nach oder gleichzeitig mit Stufe a) mit einem separaten, replizierbaren Vektor transformiert wird, der zur Expression eines vom λ-Bakteriophagen stammenden mutanten cI-Repressorgens fähig ist und der mit dem Expressionsvektor der Stufe a) voll kompatibel ist.

17. Verfahren nach einem der Ansprüche 13 bis 16, bei dem das Bakterium bei einer Temperatur von etwa 30°C bis etwa 36°C gezüchtet wird.

18. Verfahren nach Anspruch 17, bei dem unmittelbar vor Stufe b) die Temperatur der Kultur so erhöht wird. dass das temperaturempfindliche Repressorprotein inaktiviert wird.

19. Verfahren nach Anspruch 18, bei dem die Temperatur auf etwa 37°C bis 42°C echöht wird.

20. Verfahren nach einem der Ansprüche 13 bis 19, bei dem die Lyse in Stufe c) enzymatisch erfolgt.

21. Verfahren nach einem der Ansprüche 13 bis 20, bei dem das Interferon chromatographisch isoliert wird.

22. Verfahren nach einem der Ansprüche 13 bis 21, dadurch gekennzeichnet, dass die Chromatographie Antikörperaffinitätschromatographie ist.

## Claims

1. A vector capable of expressing mature human interferon containing the $P_L$ promoter and $O_L$ operator DNA sequence derived from bacteriophage λ, a DNA sequence coding for mature human interferon and a DNA sequence coding for a hybrid ribosome binding site, which is linked downstream from said $P_L$ promoter and $O_L$ operator DNA sequence and upstream from said DNA sequence coding for human interferon and which is represented by one of the following sequences:

AGGAGAATTCATG;
AGGAGAATTAATTCATG;
AGGAGAATTCTAGATG;
AGGATCTGAATTCTAGATG;
AGGAGAATTAATTCTAGATG;
AGGATCTGAATTAATTCTAGATG;
AGGAGAATTCTAGCTAGATG;
AGGATCTGAATTCTAGCTAGATG;
TTAAAAATTAAGGAGGAATTCATG and
TTAAAAATTAAGGAGGAATTAATTCATG.

2. An expression vector according to Claim 1, wherein the DNA sequence coding for mature human interferon codes for mature human immune interferon.

3. An expression vector according to Claim 2, wherein the DNA sequence coding for mature human immune interferon is represented by the general formula

(5') TGT TAC TGC CAG GAC CCA TAT GTA AAA GAA
GCA GAA AAC CTT AAG AAA TAT TTT AAT GCA GGT
CAT TCA GAT GTA GCG GAT AAT GGA ACT CTT TTC
TTA GGC ATT TTG AAG AAT TGG AAA GAG GAG AGT
GAC AGA AAA ATA ATG CAG AGC CAA ATT GTC TCC
TTT TAC TTC AAA CTT TTT AAA AAC TTT AAA GAT
GAC CAG AGC ATC CAA AAG AGT GTG GAG ACC ATC
AAG GAA GAC ATG AAT GTC AAG TTT TTC AAT AGC
AAC AAA AAG AAA CGA GAT GAC TTC GAA AAG CTG
ACT AAT TAT TCG GTA ACT GAC TTG AAT GTC CAA
CGC AAA GCA ATA CAT GAA CTC ATC CAA GTG ATG
GCT GAA CTG TCG CCA GCA GCT AAA ACA GGG AAG
CGA AAA AGG AGT CAG ATG CTG TTT CGA GGT CGA
AGA GCA TCC CAG -X (3')

wherein X is TAA, TGA or TAG.

4. An expression vector according to Claim 2, wherein the DNA sequence coding for mature human immune interferon codes for a polypeptide of the formula

EP 0 099 084 B1

H₂N- Cys-Tyr-Cys-Gln-Asp-Pro-Tyr-Val-Lys-Glu-Ala-Glu-
Asn-Leu-Lys-Lys-Tyr-Phe-Asn-Ala-Gly-His-Ser-Asp-
Val-Ala-Asp-Asn-Gly-Thr-Lei-Phe-Leu-Glv-Ile-Leu-
Lys-Asn-Trp-Lys-Glu-Glu-Ser-Asp-Arg-Lys-Ile-Met-
Gln-Ser-Gln-Ile-Val-Ser-Phe-Tyr-Phe-Lys-Leu-Phe-
Lys-Asn-Phe-Lys-Asp-Asp-Gln-Ser-Ile-Gln-Lys-Ser-
Val-Glu-Thr-Ile-Lys-Glu-Asp-Met-Asn-Val-Lys-Phe-
Phe-Asn-Ser-Asn-Lys-Lys-Lys-Arg-Asp-Asp-Phe-Glu-
Lys-Leu-Thr-Asn-Tyr-Ser-Val-Thr-Asp-Lei-Asn-Val-
Gln-Arg-Lys-Ala-Ile-His-Glu-Leu-Ile-Gln-Val-Met-
Ala-Glu-Leu-Ser-Pro-Ala-Ala-Lys-Thr-Gly-Lys-Arg-
Lys-Arg-Ser-Gln-Met-Leu-Phe-Arq-Gly-Arg-Arg-Ala-
Ser-Gln-OH.

5. An expression vector according to any one of Claims 2 - 4, wherein the DNA sequence coding for the hybrid ribosome binding site is represented by the formula

TTAAAAATTAAGGAGGAATTCATG.

6. An expression vector according to any one of Claims 2 - 4, wherein the DNA sequence coding for the hybrid ribosome binding site is represented by the formula

TTAAAAATTAAGGAGGAATTAATTCATG.

7. An expression vector according to Claim 1, wherein the DNA sequence coding for mature human interferon codes for mature human leukocyte interferon.

8. An expression vector according to Claim 7, wherein the DNA sequence coding for the hybrid ribosome binding site is represented by the formula

AGGAGAATTCATG or AGGAGAATTAATTCATG.

9. An expression vector according to Claim 1, wherein the DNA sequence coding for mature human interferon codes for mature human fibroblast interferon.

10. An expression vector according to Claim 9, wherein the DNA sequence coding for the hybrid ribosome binding site is defined by one of the following sequences:

AGGAGAATTCTAGATG;
AGGATCTGAATTCTAGATG;
AGGAGAATTAATTCTAGATG;
AGGATCTGAATTAATTCTAGATG;
AGGAGAATTCTAGCTAGATG; and
AGGATCTGAATTCTAGCTAGATG.

11. A bacterium transformed with an expression vector in accordance with any one of Claims 1 - 10.

12. A bacterium in accordance with Claim 11, which is Escherichia coli.

13. A method for the production of mature human interferon, characterized by
(a) transforming a bacterium with an expression vector in accordance with any one of Claims 1- 10;
(b) growing the transformed bacterium, whereby this produces the mature human interferon coded for by the interferon gene of an expression vector of Claims 1 to 10;
(c) lysing the transformed bacterium; and
(d) isolating the said interferon from the resulting lysate.

14. A method according to Claim 13, characterized in that the bacterium contains on its chromosome a mutant cl repressor gene which is derived from λ bacteriophage and which codes for a temperature-sensitive repressor protein.

15. A method according to Claim 13, in which the expression vector used in step (a) contains a mutant cl repressor gene which is derived from λ bacteriophage and which codes for a temperature-sensitive repressor protein.

16. A method according to Claim 13, in which the bacterium after or simultaneously with step (a) is transformed with a separate, replicable vector which is capable of expressing a mutant cl repressor gene derived from bacteriophage and which is fully compatible with the expression vector of step (a).

17. A method according to any one of Claims 13 to 16, in which the bacterium is grown at a temperature of about 30°C to about 36°C.

18. A method according to Claim 17, in which immediately prior to step (b) the temperature of the culture is raised such that the temperature-sensitive repressor protein is inactivated.

19. A method according to Claim 18, in which the temperature is raised to about 37°C to 42°C.

14

20. A method according to any one of Claims 13 to 19, in which the lysis in step (c) is effected enzymatically.
21. A method according to any one of Claims 13 to 20, in which the interferon is isolated by chromatography.
22. A method according to any one of Claims 13 to 21, characterized in that the chromatography is antibody affinity chromatography.

## Revendications

1. Vecteur apte à exprimer de l'interféron humain mature contenant la séquence d'ADN du promoteur $P_L$ et de l'opérateur $O_L$ provenant du bacteriophage $\lambda$, une séquence d'ADN codant pour l'interféron humain mature et une séquence d'ADN codant pour un site de liaison des ribosomes hybride, liant ladite séquence d'ADN du promoteur $P_L$ et de l'opérateur $O_L$ en aval et ladite séquence d'ADN codant pour l'interféron humain en amont et défini par l'une des séquences suivantes:

AGGAGAATTCATG;
AGGAGAATTAATTCATG;
AGGAGAATTCTAGATG;
AGGATCTGAATTCTAGATG;
AGGAGAATTAATTCTAGATG;
AGGATCTGAATTAATTCTAGATG;
AGGAGAATTCTAGCTAGATG;
AGGATCTGAATTCTAGCTAGATG;
TTAAAAATTAAGGAGGAATTCATG; et
TTAAAAATTAAGGAGGAATTAATTCATG.

2. Vecteur d'expression selon la revendication 1, dans lequel la séquence d'ADN codant pour l'interféron humain mature code pour l'interféron humain immun mature.

3. Vecteur d'expression selon la revendication 2, dans lequel la séquence d'ADN codant pour l'interféron humain immun mature est représentée par la formule générale

(5') TGT TAC TGC CAG GAC CCA TAT GTA AAA GAA
GCA GAA AAC CTT AAG AAA TAT TTT AAT GCA GGT
CAT TCA GAT GTA GCG GAT AAT GGA ACT CTT TTC
TTA GGC ATT TTG AAG AAT TGG AAA GAG GAG AGT
GAC AGA AAA ATA ATG CAG AGC CAA ATT GTC TCC
TTT TAC TTC AAA CTT TTT AAA AAC TTT AAA GAT
GAC CAG AGC ATC CAA AAG AGT GTG GAG ACC ATC
AAG GAA GAC ATG AAT GTC AAG TTT TTC AAT AGC
AAC AAA AAG AAA CGA GAT GAC TTC GAA AAG CTG
ACT AAT TAT TCG GTA ACT GAC TTG AAT GTC CAA
CGC AAA GCA ATA CAT GAA CTC ATC CAA GTG ATG
GCT GAA CTG TCG CCA GCA GCT AAA ACA GGG AAG
CGA AAA AGG AGT CAG ATG CTG TTT CGA GGT CGA
AGA GCA TCC CAG -X (3')

où X est TAA, TGA ou TAG.

4. Vecteur d'expression selon la revendication 2, dans lequel la séquence d'ADN codant pour l'interféron humain immun mature code pour un polypeptide de formule

$H_2N$- Cys-Tyr-Cys-Gln-Asp-Pro-Tyr-Val-Lys-Glu-Ala-Glu-
Asn-Leu-Lys-Lys-Tyr-Phe-Asn-Ala-Gly-His-Ser-Asp-
Val-Ala-Asp-Asn-Gly-Thr-Lei-Phe-Leu-Glv-Ile-Leu-
Lys-Asn-Trp-Lys-Glu-Glu-Ser-Asp-Arg-Lys-Ile-Met-
Gln-Ser-Gln-Ile-Val-Ser-Phe-Tyr-Phe-Lys-Leu-Phe-
Lys-Asn-Phe-Lys-Asp-Asp-Gln-Ser-Ile-Gln-Lys-Ser-
Val-Glu-Thr-Ile-Lys-Glu-Asp-Met-Asn-Val-Lys-Phe-
Phe-Asn-Ser-Asn-Lys-Lys-Lys-Arg-Asp-Asp-Phe-Glu-
Lys-Leu-Thr-Asn-Tyr-Ser-Val-Thr-Asp-Lei-Asn-Val-
Gln-Arg-Lys-Ala-Ile-His-Glu-Leu-Ile-Gln-Val-Met-
Ala-Glu-Leu-Ser-Pro-Ala-Ala-Lys-Thr-Gly-Lys-Arg-
Lys-ArgrSer-Gln-Met-Leu-Phe-Arq-Gly-Arg-Arg-Ala-
Ser-Gln-OH.

5. Vecteur d'expression selon l'une des revendications 2 - 4, dans lequel la séquence d'ADN codant pour le site de liaison des ribosomes hybride est représentée par la formule

TTAAAAATTAAGGAGGAATTCATG.

6. Vecteur d'expression selon l'une des revendications 2 à 4, dans lequel la séquence d'ADN codant pour le site de liaison des ribosomes hybride est représentée par la formule

TTAAAAATTAAGGAGGAATTAATTCATG.

7. Vecteur d'expression selon la revendication 1, dans lequel la séquence d'ADN codant pour l'interféron humain mature code pour l'interféron leucocytaire humain mature.

8. Vecteur d'expression selon la revendication 7, dans lequel la séquence d'ADN codant pour le site de liaison des ribosomes hybride est représentée par la formule

AGGAGAATTCATG ou AGGAGAATTAATTCATG.

9. Vecteur d'expression selon la revendication 1, dans lequel la séquence d'ADN codant pour l'interféron humain mature code pour l'interféron fibroblastique humain mature.

10. Vecteur d'expression selon la revendication 9, dans lequel la séquence d'ADN codant pour le site de liaison des ribosomes hybride est définie par l'une des séquences suivantes:

AGGAGAATTCTAGATG;
AGGATCTGAATTCTAGATG;
AGGAGAATTAATTCTAGATG;
AGGATCTGAATTAATTCTAGATG;
AGGAGAATTCTAGCTAGATG; et
AGGATCTGAATTCTAGCTAGATG.

11. Une bactérie transformée par un vecteur d'expression selon l'une des revendications 1 à 10.

12. Une bactérie selon la revendication 11, constituée par l'Escherichia coli.

13. Procédé de préparation d'interféron humain mature, caractérisé en ce que l'on
(a) transforme une bactérie par un vecteur d'expression selon l'une des revendications 1 à 10,
(b) cultive la bactérie transformée, celle-ci produisant de l'interféron humain mature codé par le gène de l'interféron d'un vecteur d'expression selon les revendications 1 à 10;
(c) lyse la bactérie transformée et
(d) isole ledit interféron du lysat obtenu.

14. Procédé selon la revendication 13, caractérisé en ce que la bactérie contient sur son chromosome un gène répresseur cI mutant provenant du bactériophage $\lambda$, codant pour une protéine du répresseur sensible à la température.

15. Procédé selon la revendication 13, dans lequel le vecteur d'expression utilisé dans l'étape (a) contient un gène répresseur cI mutant provenant du bactériophage $\lambda$, codant pour une protéine du répresseur sensible à la température.

16. Procédé selon la revendication 13, dans lequel la bactérie est transformée après ou en même temps que l'étape (a) par un vecteur séparé, pouvant être répliqué, apte à exprimer un gène répresseur cI mutant provenant du bactériophage $\lambda$ et pleinement compatible avec le vecteur d'expression de l'étape (a).

17. Procédé selon l'une des revendications 13 à 16, dans lequel la bactérie est cultivée à une température d'environ 30° C à environ 36° C.

18. Procédé selon la revendication 17, dans lequel on élève la température immédiatement avant l'étape (b), de telle manière que la protéine du répresseur sensible à la température soit inactivée.

19. Procédé selon la revendication 18, dans lequel on élève la température à environ 37° C jusqu'à 42° C.

20. Procédé selon l'une des revendications 13 à 19, dans lequel la lyse à l'étape (c) est effectuée enzymatiquement.

21. Procédé selon l'une des revendications 13 à 20, dans lequel l'interféron est isolé chromatographiquement

22. Procédé selon l'une des revendications 13 à 21, caractérisé en ce que la chromatographie est une chromatographie d'affinité d'anticorps.

EP 0 099 084 B1

Fig. 1

BamHI

HinfI

HinfI

HinfI

HpaI

N

HinfI

HinfI

HincII

BglII

P$_L$ O$_L$
1 2 3

450

350

35300
35400
35500
35600
35700
35800
35900
36000
36100
36200
36300
36400
36500

1

Fig. 2

3

Fig. 3

5

Fig. 4

$\lambda$ cI857 Sam$_7$ DNA [dam$^-$] - 49,000 bp

$\downarrow$ *Hinf* I

Isolierung der 180-220 BP-Bruchstücke

$\downarrow$ *Hae* II

Isolierung des 82 BP *Hinf* I - *Hae* II Fragments

$\downarrow$

*Hinf* I   *Mbo* I   *Hae* II

SD$_{int}$ - 82

*Mbo* I

$\downarrow$

*Hinf* I ——————— TTTTGAAGAGGATCAGAAATG ——— *Hae* II

7

Fig. 5

Fig. 6

**pRC 14**

+ *Bgl* II

+ *Eco* RI

**pRC 15**

+ *Bgl* II

+ *Eco* RI

**pRC 2**

Verknüpfung
und
Transformation

**pRC 21**

**pRC 22**

EcoRI

SD$_N$

EcoRI

SD$_{int}$

TAACTGACAGGAGAATTC

TTTTGAAGAGGATCTGAATTC

Fig. 7

P_L-250    'MODEL' RBS

Bg/II    Hae III    TTAAAAATTAAGGAGG
                    AATTTTTAATTCCTCCTTAA

Verknüpfung

+ Bg/II
+ Eco RI

Isolierung des 265 BP-Fragments

Verknüpfung
und
Transformation

Bg/II    EcoRI

Bam HI

Pst I    amp^R    tet^R

pRC 2

Bg/II    EcoRI

P_L    Bam HI

Pst I    amp^R    tet^R

pRC 23

13

Fig. 8

PRC21   pRC22

pFIF trp 69

↓ Eco RI

↓ Auffüllung mit Klenow-Polymerase I

↓ Bam HI

↓ Xba I

↓ Auffüllung mit Klenow-Polymerase I

↓ Bam HI

Verknüpfung und Transformation

pRC21/ FIF   und   pRC22/ FIF

$P_L$ → $\overset{SD_N}{\underline{AGGAG}}AATTCTAG\overline{ATG}$ — FIF

$P_L$ → $\overset{SD_{int}}{\underline{AGGATCTG}}AATTCTAG\overline{ATG}$ — FIF

15

Fig. 9

Fig. 10

```
                                          S1
5'ACTTCTTTGGCTTAATTCTCTCGGAAACG ATG AAA TAT ACA AGT TAT ATC TTG
                                  Met Lys Tyr Thr Ser Tyr Ile Leu

                                      S20  1
GCT TTT CAG CTC TGC ATC GTT TTG GGT TCT CTT GGC TGT TAC TGC CAG
Ala Phe Gln Leu Cys Ile Val Leu Gly Ser Leu Gly Cys Tyr Cys Gln
                                                              20
GAC CCA TAT GTA AAA GAA GCA GAA AAC CTT AAG AAA TAT TTT AAT GCA
Asp Pro Tyr Val Lys Glu Ala Glu Asn Leu Lys Lys Tyr Phe Asn Ala

GGT CAT TCA GAT GTA GCG GAT AAT GGA ACT CTT TTC TTA GGC ATT TTG
Gly His Ser Asp Val Ala Asp Asn Gly Thr Leu Phe Leu Gly Ile Leu
            40
AAG AAT TGG AAA GAG GAG AGT GAC AGA AAA ATA ATG CAG AGC CAA ATT
Lys Asn Trp Lys Glu Glu Ser Asp Arg Lys Ile Met Gln Ser Gln Ile
                                60
GTC TCC TTT TAC TTC AAA CTT TTT AAA AAC TTT AAA GAT GAC CAG AGC
Val Ser Phe Tyr Phe Lys Leu Phe Lys Asn Phe Lys Asp Asp Gln Ser
                                              80
ATC CAA AAG AGT GTG GAG ACC ATC AAG GAA GAC ATG AAT GTC AAG TTT
Ile Gln Lys Ser Val Glu Thr Ile Lys Glu Asp Met Asn Val Lys Phe
                                                              100
TTC AAT AGC AAC AAA AAG AAA CGA GAT GAC TTC GAA AAG CTG ACT AAT
Phe Asn Ser Asn Lys Lys Lys Arg Asp Asp Phe Glu Lys Leu Thr Asn

TAT TCG GTA ACT GAC TTG AAT GTC CAA CGC AAA GCA ATA CAT GAA CTC
Tyr Ser Val Thr Asp Leu Asn Val Gln Arg Lys Ala Ile His Glu Leu
            120
ATC CAA GTG ATG GCT GAA CTG TCG CCA GCA GCT AAA ACA GGG AAG CGA
Ile Gln Val Met Ala Glu Leu Ser Pro Ala Ala Lys Thr Gly Lys Arg
                                140                       146
AAA AGG AGT CAG ATG CTG TTT CGA GGT CGA AGA GCA TCC CAG TAA TGG
Lys Arg Ser Gln Met Leu Phe Arg Gly Arg Arg Ala Ser Gln ---

TTGTCCTGCCTGCAATATTTGAATTTTAAATCTAAATCTATTTATTAATATTTAACATTATTT

ATATGGGGAATATATTTTTAGACTCATCAATCAAATAAGTATTTATAATAGCAACTTTTGTGT

AATGAAAATGAATATCTATTAATATATGTATTATTTATAATTCCTATATCCTGTGACTGTCTC

ACTTAATCCTTTGTTTTCTGACTAATTAGGCAAGGCTATGTGATTACAAGGCTTTATCTCAGG

GGCCAACTAGGCAGCCAACCTAAGCAAGATCCCATGGGTTGTGTGTTTATTTCACTTGATGAT

ACAATGAACACTTATAAGTGAAGTGATACTATCCAGTTACTGCCGGTTTGAAAATATGCCTGC

AATCTGAGCCAGTGCTTTAATGGCATGTCAGACAGAACTTGAATGTGTCAGGTGACCCTGATG

AAAACATAGCATCTCAGGAGATTTCATGCCTGGTGCTTCCAAATATTGTTGACAACTGTGACT

GTACCCAAATGGAAAGTAACTCATTTGTTAAAATTATCAATATCTAATATATATGAATAAAGT

C 3'
```

Fig. 11

γ-IF-Kodier-
bereich

*Bst* NI

A

A

*Bst* NI

900 bp *Bst* NI-Fragment von pHIT 3709

Verknüpfung mit synthetischem Oligonukleotid:

*Bst* NI

| Met | 1 | 2 | 3 |
|-----|---|---|---|

AATTC ATG TGT TAT TGT C
    G TAC ACA ATA ACA GT

*Eco* RI

*Bgl* II    *Eco* RI

P_L

*Eco* RI

*Pst* I    amp^R    tet^R    *Bam* HI

pRC 22

*Bgl* II    *Eco* RI    T-IF

P_L

*Bgl* I

amp^R

*Eco* RI

tet^R

pRC 22 /IFI-900

*Eco* RI

P_L →    SD_int    AGGATCTGAATTC    Met 1 2 3 4    ATG TGT TAT TGT CAG    —    T-IF

Fig. 12

```
EcoRI
 ↓
PL ──▷ TTAAAAATTAAGGAGGAATTCATG ...... 𝝉-IF
              │ +EcoRI
              ▽
              │ Auffüllung mit Klenow-Polymerase I
              ▽
              │ Verknüpfung der stumpfen Enden
              ▽
PL ──▶ TTAAAAATTAAGGAGGAATTAATTCATG ...... 𝝉-IF
```

pRC 231/ IFI - 900

23